(19) Europäisches Patentamt
European Patent Office
Office européen des brevets

(11) Publication number: **0 033 580**
**B1**

(12) # EUROPEAN PATENT SPECIFICATION

(45) Date of publication of patent specification: **25.07.84**

(21) Application number: **81300056.9**

(22) Date of filing: **07.01.81**

(51) Int. Cl.³: **C 07 C 93/14,**
C 07 C 87/60,
C 07 C 87/62,
C 07 C 121/52,
C 07 C 101/52,
C 07 C 101/60,
A 61 K 31/085,
A 01 N 33/06, A 01 N 33/18

(54) **Novel benzenamines, their preparation and fungicide and anticoccidial compositions containing them.**

(30) Priority: **08.01.80 US 110307**
**08.01.80 US 110308**

(43) Date of publication of application:
**12.08.81 Bulletin 81/32**

(45) Publication of the grant of the patent:
**25.07.84 Bulletin 84/30**

(84) Designated Contracting States:
**AT BE CH DE FR GB IT LI LU NL SE**

(56) References cited:
**EP - A - 0 000 156**
**EP - A - 0 003 430**
**EP - A - 0 004 642**
**GB - A - 1 320 648**

**The file contains technical information submitted after the application was filed and not included in this specification**

(73) Proprietor: **ELI LILLY AND COMPANY**
**307, East McCarty Street**
**Indianapolis Indiana 46285 (US)**

(72) Inventor: **Clinton, Albert James**
**deceased (US)**

(74) Representative: **Hudson, Christopher Mark et al,**
**Erl Wood Manor**
**Windlesham Surrey GU20, 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Benzenamines have experienced widespread usage in the field of agriculture. Numerous benzenamines are known which are effective as herbicidal agents. Among the most widely used herbicidal benzenamines are trifluralin, which is N,N-di-*n*-propyl-2,6-dinitro-4-trifluoromethylbenzenamine, butralin, which is N-(1-methylpropyl)-2,6-dinitro-4-*tert.*-butylbenzenamine, and benefin, N-butyl-N-ethyl-2,6-dinitro-4-trifluoromethylbenzenamine.

Several benzenamines are known to be useful in animal health. For example, European Patent No. 156, published January 10, 1979, discloses various N-(2,4-dinitro-6-trifluoromethylphenyl)benzeamines which are said to exhibit useful insecticidal, acaricidal, nematocidal, insect growth retardant, fungicidal and bactericidal activity. Similary, antifungal benzeneamines are disclosed in U.S. Patent No. 4,152,460 and in EP-A-0 004 642.

This invention provides certain novel N-(nitrophenyl)-polyfluoroethoxybenzenamines which are potent antifungal agents, and which also display ectoparasitic and anticoccidial activity.

This invention concerns novel benzenamines, formulations containing such compounds, and a method for treating soil and plant fungal diseases. The invention is more particularly directed to N-(nitrophenyl)-(tetra and penta fluoroethoxy)benzenamine compounds of the formula

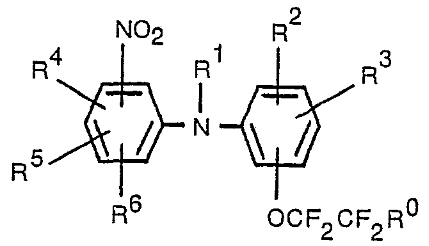

I

wherein:

$R^0$ is hydrogen or fluoro;

$R^1$ is hydrogen or $C_1$—$C_2$ alkyl;

$R^2$ and $R^3$ independently are hydrogen or halo;

$R^4$ is hydrogen, trifluoromethyl, cyano, $C_1$—$C_4$ alkyl, hydroxycarbonyl or $C_1$—$C_4$ alkoxycarbonyl;

$R^5$ is hydrogen, halo, nitro, hydroxy, methoxy or amino;

$R^6$ is hydrogen or nitro; and the physiologically-acceptable salts thereof.

Preferred compounds provided by this invention include those having the above formula wherein $R^6$ is nitro.

Expecially preferred compounds comprehended by this invention are those of the formula

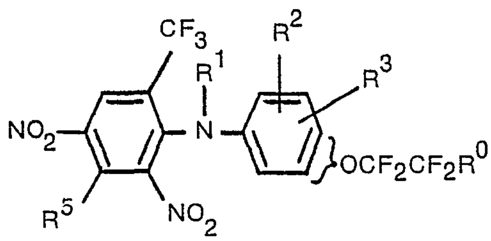

II

wherein:

$R^0$ is hydrogen or fluoro;

$R^1$ is hydrogen or $C_1$—$C_2$ alkyl;

$R^2$ and $R^3$ independently are hydrogen or halo;

$R^5$ is hydrogen, or halo; and the physiologically-acceptable salts thereof.

Additionally preferred compounds are those wherein $R^2$ and $R^3$ are the same, $R^4$ is hydrogen, trifluoromethyl, cyano, $C_1$—$C_4$ alkyl, or hydroxycarbonyl; $R^5$ is chloro, bromo or nitro; and $R^6$ is nitro.

Another preferred group of benzenamines according to this invention have the above formula wherein $R^0$, $R^1$, $R^2$, $R^3$ and $R^5$ all are hydrogen, $R^4$ is trifluoromethyl and $R^6$ is nitro.

A further embodiment of this invention is formulation comprising an N-(nitrophenyl)-polyfluoroethoxybenzenamine defined by the above general formula admixed with a suitable carrier, diluent or excipient therefor.

Also provided by this invention is a method for treating fungicidal infections in plants and in soil comprising applying to the locus to be treated an antifungal amount of a benzenamine defined by the above formula.

$R^1$ in the above formula defines hydrogen or $C_1$—$C_2$ alkyl, namely methyl or ethyl.

$R^2$, $R^3$ and $R^5$ include the groups referred to herein as "halo". The term bears its art-recognized meaning of fluoro, chloro, bromo and iodo. Preferred halo groups defined by $R^2$, $R^3$ and $R^5$ are chloro and bromo.

$R^4$ in the above formula includes $C_1$—$C_4$ alkyl groups, which are straight and branched chain alkyl group having from one to four carbon atoms. Typical $C_1$—$C_4$ alkyl groups include methyl, ethyl, *n*-propyl *iso*-propyl, *n*-butyl and *tert.*-butyl.

$R^4$ additionally defines a $C_1$—$C_4$ alkoxycarbonyl moiety, for instance methoxycarbonyl, ethoxycarbonyl, *iso*propoxycarbonyl, *n*-butoxycarbonyl, and related groups.

The N-nitrophenyl-(tetra and penta)fluoroethoxybenzenamines comprehended by this invention can be prepared by any of several chemical processes. A preferred and commonly utilized process involves the condensation reaction of a substituted phenyl electrohilic agent with a phenylamine derivative. For example, a polyfluoroethoxyphenyl electrophilic agent such as a phenyl halide can be condensed with a nitrophenylamine according to the following scheme:

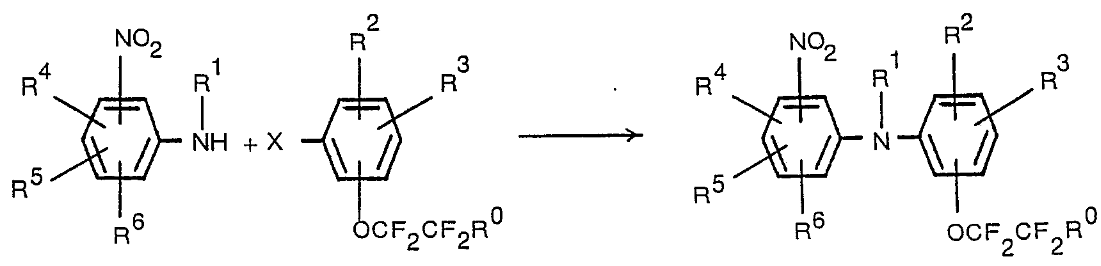

wherein $R^0$, $R^1$, $R^2$, $R^3$, $R^4$, $R^5$ and $R^6$ are as defined above, and X is a good leaving group such as halo, for instance chloro, bromo or iodo. A similar, yet alternative, process comprises condensing a nitrophenyl electrophilic agent with a polyfluoroethoxyphenylamine according to the following scheme:

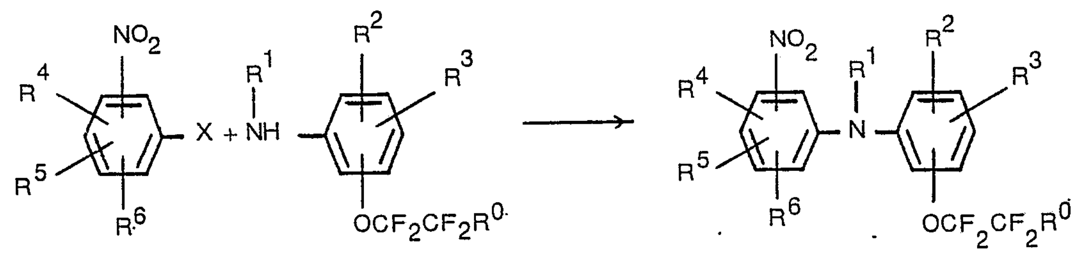

wherien $R^0$, $R^1$, $R^2$, $R^3$, $R^4$, $R^5$ and $R^6$ are as defined above, and X is a good leaving group such as halo. Such reactions are preferably carried out when $R^1$ is hydrogen.

According to the processes outlined above, a substituted phenyl electrophilic agent such as a tetra or pentafluoroethoxyphenyl chloride is mixed with about an equimolar quantity of a nitrophenylamine. The condensation reaction generally is carried out in an unreactive organic solvent and in the presence of a strong base. Commonly used unreactive organic solvents include amides, for instance dimethylformamide or hexamethylphosphortriamide; ethers such as tetrahydrofuran, diethyl ether, or dioxane; sulfoxides such as dimethyl sulfoxide; alcohols such as methanol or ethanol; and related solvents. Strong bases which may be utilized in the reaction include alkali metal hydrides, for instance sodium hydride or lithium hydride; amines such as triethylamine, pyridine, DBN (1,5-diazabicyclo [4.3.0]-non-5-ene) and DBU (1,5-diazabicyclo-[5.4.0] undec-5-ene, carbonates such as potassium carbonate and sodium carbonate; alkoxides such as potassium *tert.*-butoxide, and the like.

The condensation reaction generally is carried out by first adding a phenylamine to a strong base in a suitable solvent. For instance, a phenylamine such as 3-(1,1,2,2-tetrafluoroethoxyphenyl)amine can be reacted with a base such as sodium hydride in a solvent such as dimethylformamide. The reactants can be employed in about equimolar quantities, or if desired an excess of base, for example about a 0.1 to about a 10 molar excess, can be utilized if desired. The phenylamine and the strong base generally are allowed to react for up to about 3 hours at a temperature of about —30° to about 30°C., preferably about 0° to about 25°C. Following the initial reaction of the phenylamine and the strong base, the desired substituted phenyl electrophilic agent, for instance a compound such as 2,4-dinitro-6-trifluoromethylphenyl chloride, is added to the reaction mixture, and the reaction is permitted to continue for about 2 to about 48 hours at a temperature of about 0° to about 100°C.

The product of the condensation reaction is a compound comprehended by this invention and is readily isolated by simply adding the reaction mixture to an aqueous acid solution, for instance dilute aqueous hydrochloric acid or sulfuric acid. The desired product often precipitates out of the aqueous acid solution as a solid or an oil. Alternatively, the product may be extracted into a water immiscible organic solvent such as diethyl ether, ethyl acetate, dichloromethane, or the like. Removal of the organic solvent, for instance by evaporation under reduced pressure, then provides a compound of this invention. The product thus formed can be further purified if desired by any of several standard methods, including column chromatography over a solid support such as silica gel or the like, or

crystrallization from common solvents such as ethanol, benzene, skelly B, diethyl ether, acetone, and the like.

Certain benzenamines provided by this invention can be prepared by modification of an existing benzenamine prepared as described above. For example, N-alkylation of a benzenamine having the above formula wherein $R^1$ is hydrogen affords the corresponding benzenamine wherein $R^1$ is $C_1$—$C_2$ alkyl. Such alkylation reactions typically are accomplished by combining an alkylating agent with a benzenamine (wherein $R^1$ is hydrogen) in an unreactive organic solvent and in the presence of a base. Typical alkylating agents commonly used include alkyl halides such as methyl bromide or ethyl iodide, as well as sulfates such as dimethyl sulfate and diethyl sulfate. Commonly used solvents include acetone, benzene, methyl ethyl ketone, dimethylsulfoxide and the like. The alkylation routinely is substantially complete within about two to about seventy-two hours when carried out at a temperature of about 30 to about 150°C. The N-alkylated benzenamine is generally isolated by extraction of the reaction mixture with a solvent such as diethyl ether or benzene, and then evaporation of the solvent from the extract. The product can be further purified if desired by crystallization, chromatography, distillation, or related purification techniques.

Benzenamines bearing a carboxylic acid moiety, that is to say compounds of the above general formula wherein $R^4$ is hydroxycarbonyl, are readily esterified to provide benzenamines wherein $R^4$ is a $C_1$—$C_4$ alkoxy carbonyl group. Such conversion can be accomplished by standard esterification reactions. Methyl esters are often preferably prepared by simply reacting a free acid with diazomethane in a suitable solvent such as diethyl ether. Esterification can also be accomplished by condensing a benzenamine carboxylic acid with a $C_1$—$C_4$ alkanol in the presence of an acid, for instance sulfuric acid or the like. Alternatively, a benzenamine carboxylic acid can be converted to an acid halide, and the acid halide then can be condensed with a $C_1$—$C_4$ alkanol. For example, reaction of a benzenamine such as N-(2-nitro-4-hydroxycarbonylphenyl)-4-(1,1,2,2-tetrafluoroethoxy)benzenamine with oxalyl chloride affords N-(2-nitro-4-chlorocarbonylphenyl)-4-(1,1,2,2-tetrafluoroethoxy)-benzenamine, which was reacted with an alkanol such as *iso*propanol affords N-(2-nitro-4-*iso*propoxy-carbonylphenyl)-4-(1,1,2,2-tetrafluoroethoxy)benzenamine.

Benzenamines having the above formula wherein one or both of $R^2$ and $R^3$ are hydrogen can be halogenated by reaction with a halogenating agent in a suitable solvent. For instance, a compound such as N-(2,6-dinitro-4-trifluoromethyl)-3-(1,1,2,2-tetrafluoroethoxy)benzenamine can be reacted with about one equivalent, or an excess if desired, of bromine in the presence of a solvent such as dichloromethane or a mixture of acetic acid and water. Such reaction effects bromination to provide, for instance. N-(2,6-dinitro-4-trifluoromethyl)-2,4-dibromo-5-(1,1,2,2-tetrafluoroethoxy)benzenamine.

When desired, the above described halogenation reaction can be carried out utilizing less than one molar quantity of halogenating agent, thereby effecting monohalogenation instead of dihalogenation. The monohalogenation product can be further halogenated if desired with the same or a different halogenating agent. For instance a benzenamine such as N-(2,6-dinitro-4-trifluoromethylphenyl)-3-(1,1,2,2,2-pentafluoroethoxy)benzenamine can be reacted with about a 0.5 molar amount of chloride in dichloromethane at about 25°C. to give N-(2,6-dinitro-4-trifluoromethylphenyl)-2-chloro-3-(1,1,2,2,2-pentafluoroethoxy)benzenamine. The latter compound can be further halogenated, for instance by reaction with about a 0.5 to 10, molar amount of bromine in dichloromethane, to provide the corresponding dihalogenated derivative, for example N-(2,6-dinitro-4-trifluoromethylphenyl)-2-chloro-4-bromo-5-(1,1,2,2,2-pentafluoroethoxy)benzenamine.

The benzenamines contemplated herein wherein $R^1$ is hydrogen are weakly acidic in nature by virtue of the activated proton attached to the amino nitrogen atom to which the two aromatic rings are attached. Because of this acidic nature, the benzenamines readily form physiologically-acceptable salts by reaction with any of a number of common inorganic and organic bases. The salts are in general solids and thus lend themselves to purification by crystallization from common solvents such as ethanol, acetone, ethyl acetate, methyl ethyl ketone, and the like.

The salts provided herein are prepared by reaction of about equimolar quantities of a benzenamine and a base. Inorganic bases commonly employed to form salts of this invention include the alkali metal hydroxides such as sodium hydroxide and potassium hydroxide, as well as alkali metal amides such as lithium amide and potassium amide. Routinely used organic bases include alkali metal alkoxides such as potassium *tert.*-butoxide and sodium methoxide, as well as alkali metal amides such as lithium or potassium diisopropylamide.

When it is desired to regenerate the free amine from an addition salt, the salt is simply reacted with an acid such as hydrochloric acid or sulfuric acid. For example, reaction of the sodium salt of N-(2,4-dinitrophenyl)-3-(1,1,2,2-tetrafluoroethoxy)benzenamine with about one equivalent amount of hydrochloric acid converts the salt to a free amine to provide N-(2,4-dinitrophenyl)-3-(1,1,2,2-tetrafluoroethoxy)benzenamine.

Representative benzenamines comprehended by this invention include the following:

N-(2,6-dinitro-4-trifluoromethylphenyl-3 -(1,1,2,2-tetrafluoroethoxy)benzenamine;
N-(2,6-dinitro-4-cyanophenyl)-2 -(1,1,2,2-tetrafluoroethoxy)benzenamine;
N-(2,4-dinitro-6-trifluoromethylphenyl)-N-ethyl-4-(1,1,2,2 -tetrafluoroethoxy)benzenamine;
N-(2,4-dinitro-6-hydroxycarbonylphenyl)-3-(1,1,2,2-tetrafluoroethoxy)-4-chlorobenzenamine;

N-(3-nitro-4-chlorophenyl)-N-methyl-4-(1,1,2,2-tetrafluoroethoxy)benzenamine;
N-(2-trifluoromethyl-4-nitrophenyl-2-(1,1,2,2-tetrafluoroethoxy)benzenamine;
N-(3-trifluoromethyl-4-nitrophenyl)-N-methyl-3-(1,1,2,2-tetrafluoroethoxy)benzenamine;
N-(2,6-dinitro-4-ethoxycarbonylphenyl-3-(1,1,2,2-tetrafluoroethoxy)-4,5-dichlorobenzenamine;
N-(2-nitrophenyl)-3-(1,1,2,2-tetrafluoroethoxy)benzenamine;
N-(2,4-dinitro-6-*iso*propyl)-N-ethyl-2,6-dichloro-4-(1,1,2,2-tetrafluoroethoxy)benzenamine;
N-(2,4,6-trinitrophenyl)-N-ethyl-3-(1,1,2,2-tetrafluoroethoxy)benzenamine;
N-(2,6-dinitro-3-bromo-4-trifluoromethylphenyl)-4-(1,1,2,2,2-pentafluoroethoxy)benzenamine;
N-(2-nitro-4-*tert*.-butylphenyl)-2,6-dibromo-4-(1,1,2,2-tetrafluoroethoxy)benzenamine;
N-(3,4-dinitrophenyl)-2-bromo-6-chloro-4-(1,1,2,2,2-pentafluoroethoxy)benzenamine;
N-(2,3,4-trinitro-5-methylphenyl)-2 -(1,1,2,2-tetra-fluoroethoxy)-3,5-dibromobenzenamine;
N-(2-nitro-4-ethoxycarbonylphenyl)-N-ethyl-3-(1,1,2,2-tetrafluoroethoxy)benzenamine;
N-(2,4-dinitro-6-*n*-butylphenyl)-2,6-dichloro-3-(1,1,2,2-tetrafluoroethoxy)benzenamine;
N-(2,6-dinitro-4-hydroxycarbonylphenyl)-3-(1,1,2,2-tetrafluoroethoxy)benzenamine;
N-(2,4-dinitro-6-hydroxycarbonylphenyl)-2,6-difluoro-4-(1,1,2,2-tetrafluoro)benzenamine;
N-(2,4,6-trinitrophenyl)-N-methyl-2,4-diiodo-5-(1,1,2,2-pentafluoroethoxy)benzenamine;
Sodium N-(2-nitrophenyl)-3-(1,1,2,2,2-pentafluoroethoxy)benzenamide;
Potassium N-(2,6-dinitro-4-cyanophenyl)-2,6-dibromo-3-(1,1,2,2 -tetrafluoroethoxy)benzenamide;
Lithium N-(2,4-dinitro-6-trifluoromethylphenyl)-3-(1,1,2,2 -tetrafluoroethoxy)benzenamide;
Sodium N-(2,6-dinitro-4-trifluoromethylphenyl)-4-(1,1,2,2 -tetrafluoroethoxy)benzenamide;
Lithium N-(2,4,6-trinitrophenyl)-2-(1,1,2,2-tetrafluoroethoxy)benzenamide;
N-(2-trifluoromethyl-5-chloro-4,6-dinitrophenyl)-4-(1,1,2,2-tetrafluoroethoxy)benzenamine;
N-(2,4-dinitro-6-hydroxycarbonylphenyl)-2,4-dibromo-5-(1,1,2,2-tetrafluoroethoxy)benzenamine;
Potassium N-(2,6-dinitro-4-trifluoromethyl)-3-bromo-4-(1,1,2,2-tetrafluoroethoxy)benzenamide;
Sodium N-(2-nitro-3-chloro-4-cyanophenyl)-2-(1,1,2,2-tetrafluoroethoxy)benzenamide;
N-(2,6-dinitro-4 -trifluoromethylphenyl)-2,4-dichloro-5-(1,1,2,2,2-pentafluoroethoxy)benzenamine;
N-(2,4,6-trinitrophenyl)-3-(1,1,2,2,2-pentafluoroethoxy)benzenamine;
N-(2-trifluoromethyl-4-nitrophenyl)-3-(1,1,2,2,2-pentafluoroethoxy)benzenamine;
N-(2,4-dinitro-6-trifluoromethylphenyl)-N-ethyl-4-(1,1,2,2,2-pentafluoroethoxy)benzenamine; and the like.

The detailed examples which follow illustrate the preparation of representative compounds embraced by the present invention. The examples are representative only and should not be construed as limiting in any respect.

## Example 1

N-(2,4-dinitro-6-trifluoromethylphenyl)-4-(1,1,2,2-tetrafluoroethoxy)benzenamine.

To a stirred solution of 1.0 g. of 4-(1,1,2,2-tetrafluoroethoxy)phenylamine in 50 ml. of ethanol containing 3.0 ml. of triethylamine were added in one portion 1.3 g. of 2,4-dinitro-6-trifluoromethylphenyl chloride. The reaction mixture was heated at reflux for sixteen hours following the addition. The reaction mixture then was added to 100 ml. of ice water containing about 10 ml. of hydrochloric acid. The precipitate which formed was collected and dissolved in diethyl ether. The ethereal solution was washed with water and dried, and the solvent was removed by evaporation under reduced pressure to provide 1.2 g. of N-(2,4-dinitro-6-trifluoromethylphenyl)-4 -(1,1,2,2-tetrafluoroethoxy)benzenamine.

Yield 54.9% M.P. 105—107°C.
Analysis calculated for $C_{15}H_8F_7N_3O_5$

| | | | |
|---|---|---|---|
| Theory: | C, 40.65; | H, 1.82; | N, 9.48 |
| Found: | C, 40.89; | H, 2.08; | N, 9.66 |

## Example 2

N-(2,6-dinitro-4-*tert*.-butylphenyl)-4-(1,1,2,2-tetrafluoroethoxy)benzenamine.

A solution of 2.5 g. of 4-(1,1,2,2-tetrafluoroethoxy)phenylamine hydrochloride in 30 ml. of ethanol containing 5.0 ml. of triethylamine and 2.6 g. of 2,6-dinitro-4-*tert*.-butylphenyl chloride was stirred and heated at reflux for sixteen hours. The reaction mixture was then slowly added to 100 ml. of ice water containing 10 ml. of concentrated hydrochloric acid, and the aqueous mixture was stirred for thirty minutes. The oil which formed was collected and crystallized from skelly-B solvent and diethyl ether to give 2.4 g. of N-(2,6-dinitro-4-*tert*.-butylphenyl)-4-(1,1,2,2 -tetrafluoroethoxy)benzenamine.

Yield 51.3% M.P. 142—143°C.
Analysis calculated for $C_{18}H_{17}F_4N_3O_5$

| | | | |
|---|---|---|---|
| Theory: | C, 50.12; | H, 3.97; | N, 9.74 |
| Found: | C, 50.38; | H, 4.02; | N, 9.99 |

## Examples 3—12

Following the general procedure of Examples 1 and 2, the appropriate fluoroethoxyphenylamine

was reacted with the appropriate nitrophenyl halide in the presence of triethylamine in ethanol to give the following benzenamines.

N-(2,6-dinitro-4-cyanophenyl)-4-(1,1,2,2-tetrafluoroethoxy)benzenamine.

Yield 89.3% M.P. 104—106°C.
Analysis calculated for $C_{15}H_8F_4N_4O_5$

| | | | |
|---|---|---|---|
| Theory: | C, 45.01; | H, 2.01; | N, 14.00 |
| Found: | C, 45.29; | H, 1.95; | N, 14.04 |

N-(2,6-dinitro-4-hydroxycarbonylphenyl)-4-(1,1,2,2-tetrafluoroethoxy)benzenamine.

Yield 74.6% M.P. 234—236°C.
Analysis calculated for $C_{15}H_{11}N_3O_7$

| | | | |
|---|---|---|---|
| Theory: | C, 42.97; | H, 2.16; | N, 10.02 |
| Found: | C, 43.25; | H, 2.36; | N, 10.10 |

N-(2,6-dinitro-3-chloro-4-trifluoromethylphenyl)-4-(1,1,2,2-tetrafluoroethoxy)benzenamine.

Yield 60.3% M.P. 127—130°C.
Analysis calculated for $C_{15}H_7ClF_7N_3O_5$

| | | | |
|---|---|---|---|
| Theory: | C, 37.70; | H, 1.47; | N, 8.80 |
| Found: | C, 37.95; | H, 1.53; | N, 8.59 |

N-(2,4-dinitro-6-hydroxycarbonylphenyl)-4-(1,1,2,2-tetrafluoroethoxy)benzenamine.

Yield 50.0% M.P. 192—195°C.
Analysis calculated for $C_{15}H_8F_4N_3O_7$

| | | | |
|---|---|---|---|
| Theory: | C, 42.97; | H, 2.16; | N, 10.02 |
| Found: | C, 43.23; | H, 2.30; | N, 9.74 |

N-(2,6-dinitro-4-*tert.*-butylphenyl)-3-(1,1,2,2-tetrafluoroethoxy)benzenamine.

M.P. 129—131°C.
Analysis calculated for $C_{18}H_{17}F_4N_3O_5$

| | | | |
|---|---|---|---|
| Theory: | C, 50.12; | H, 3.97; | N, 9.74 |
| Found: | C, 50.02; | H, 3.89; | N, 9.48 |

N-(2,6-dinitro-4-trifluoromethylphenyl)-3-(1,1,2,2-tetrafluoroethoxy)benzenamine. (oil)

Analysis calculated for $C_{15}H_8F_7N_3O_5$

| | | | |
|---|---|---|---|
| Theory: | C, 40.65; | H, 1.82; | N, 9.48 |
| Found: | C, 40.91; | H, 1.84; | N, 9.24 |

N-(2,4-dinitro-6-trifluoromethylphenyl)-3-(1,1,2,2-tetrafluoroethoxy)benzenamine.

M.P. 74—76°C.
Analysis calculated for $C_{15}H_7F_7N_3O_5$

| | | | |
|---|---|---|---|
| Theory: | C, 40.56; | H, 1.82; | N, 9.48 |
| Found: | C, 40.87; | H, 2.02; | N, 9.49 |

N-(2,6-dinitro-4-trifluoromethylphenyl)-4-(1,1,2,2-tetrafluoroethoxy)benzenamine.

Yield 83.5% M.P. 102—105°C.
Analysis calculated for $C_{15}H_8F_7N_3O_5$

| | | | |
|---|---|---|---|
| Theory: | C, 40.65; | H, 1.82; | N, 9.48 |
| Found: | C, 40.82; | H, 1.79; | N, 9.63 |

N-(2,4,6-trinitrophenyl)-4-(1,1,2,2-tetrafluoroethoxy)benzenamine.

M.P. 141—142°C.
Analysis calculated for $C_{14}H_8N_4O_7$

| | | | |
|---|---|---|---|
| Theory: | C, 40.01; | H, 1.92; | N, 13.33 |
| Found: | C, 40.17; | H, 2.04; | N, 13.09 |

N-(2,4,6-trinitrophenyl)-3-(1,1,2,2-tetrafluoroethoxy)benzenamine.

M.P. 128—129°C.
Analysis calculated for $C_{14}H_8N_4O_7$

| | | | |
|---|---|---|---|
| Theory: | C, 40.01; | H, 1.92; | N, 13.33 |
| Found: | C, 40.14; | H, 1.99; | N, 13.44 |

Example 13

N-(2-trifluoromethyl-4-nitrophenyl)-3-(1,1,2,2-tetrafluoroethoxy)benzenamine.

Two grams of a 50% suspension of sodium hydride in mineral oil were washed several times with pentane, and then suspended in 25 ml. of N,N-dimethylformamide. To the stirred mixture were added in one portion 3.8 g. of 3-(1,1,2,2-tetrafluoroethoxy)phenylamine. The reaction mixture was stirred at ambient temperature for thirty minutes, and then 4.5 g of 2-trifluoromethyl-4-nitrophenylchloride were added dropwise over five minutes to the reaction mixture. The mixture was stirred for three hours at room temperature, and then added slowly to 100 ml. of dilute hydrochloric acid solution. An oil which precipitated was collected, dissolved in diethyl ether, and chromatographed over silica gel. After collecting the fractions shown by thin layer chromatography to contain a single product and evaporating the solvent therefrom, there were recovered, as an oil, 2.0 g. of N-(2-trifluoromethyl-4-nitrophenyl)-3-(1,1,2,2-tetrafluoroethoxy)benzenamine.

Analysis calculated for $C_{15}H_9F_7N_3O_3$

| | | | |
|---|---|---|---|
| Theory: | C, 45.24; | H, 2.28; | N, 7.03 |
| Found: | C, 45.03; | H, 2.24; | N, 7.09 |

Examples 14—17

N-(2,6-dinitro-4-trifluoromethylphenyl)-2-(1,1,2,2-tetrafluoroethoxy)benzenamine.

Analysis calculated for $C_{15}H_8F_7N_3O_5$

| | | | |
|---|---|---|---|
| Theory: | C, 40.65; | H, 1.82; | N, 9.48 |
| Found: | C, 40.85; | H, 1.80; | N, 9.48 |

N-(2,4-dinitro-6-hydroxycarbonylphenyl)-2-(1,1,2,2-tetrafluoroethoxy)benzenamine.

Analysis calculated for $C_{15}H_9F_4N_3O_7$

| | | | |
|---|---|---|---|
| Theory: | C, 43.08; | H, 1.93; | N, 10.05 |
| Found: | C, 43.27; | H, 2.01; | N, 9.81 |

N-(2,4,6-trinitrophenyl)-2-(1,1,2,2-tetrafluoroethoxy)benzenamine.
M.P. 114—115°C.
Analysis calculated for $C_{14}H_8F_4N_4O_7$

| | | | |
|---|---|---|---|
| Theory: | C, 40.01; | H, 1.92; | N, 13.33 |
| Found: | C, 39.88; | H, 1.99; | N, 13.62 |

N-(2,4-dinitro-6-*tert.*-butylphenyl)-4-(1,1,2,2-tetrafluoroethoxy)benzenamine.

M.P. 98—101°C.
Analysis calculated for $C_{18}H_{17}F_4N_3O_5$

| | | | |
|---|---|---|---|
| Theory: | C, 50.12; | H, 3.97; | N, 9.74 |
| Found: | C, 50.24; | H, 4.01; | N, 9.86 |

Example 18

N-(2,4-dinitro-6-trifluoromethylphenyl)-N-methyl-3-(1,1,2,2-tetrafluoroethoxy)benzenamine.

A solution of 4.0 ml. of dimethylsulfate in 30 ml. of acetone containing 5.0 g. of sodium carbonate and 3.0 g. of N-(2,4-dinitro-6-trifluoromethylphenyl-3-(1,1,2,2-tetrafluoroethoxy)benzenamine was heated at reflux for sixteen hours. An additional 2.0 ml. of dimethyl sulfate was added to the reaction mixture, and refluxing was continued for an additional four hours. The reaction mixture was cooled to room temperature and diluted by the addition of 150 ml. of water. The aqueous mixture was stirred for thirty minutes, and then the product was extracted into diethyl ether. The ethereal extracts were combined, washed with water, dried, and the solvent was removed by evaporation under reduced pressure to provide 3.7 g. of an oil, identified as N-(2,4-dinitro-6-trifluoromethylphenyl)-N-methyl-3-(1,1,2,2-tetrafluoroethoxy)benzenamine.

Analysis calculated for $C_{16}H_7F_7N_3O_5$

| | | | |
|---|---|---|---|
| Theory: | C, 42.03; | H, 2.20; | N, 9.19 |
| Found: | C, 42.24; | H, 2.34; | N, 9.32 |

Example 19

Following the procedure of Example 18, N-(2,4-dinitro-6-trifluoromethylphenyl)-4-(1,1,2,2-tetrafluoroethoxy)benzenamine was reacted with dimethyl sulfate and sodium carbonate in acetone to provide N-(2,4-dinitro-6-trifluoromethylphenyl)-N-methyl-4-(1,1,2,2-tetrafluoroethoxy)benzenamine.

M.P. 91—92°C.
Analysis calculated for $C_{16}H_7F_7N_3O_5$
Theory: C, 42.03; H, 2.20; N, 9.19
Found: C, 42.27; H, 2.06; N, 9.43

Example 20

N-(2,4-dinitro-6-trifluoromethylphenyl)-N-methyl-2,4-dibromo-5-(1,1,2,2-tetrafluoroethoxy)-benzenamine.

To a stirred solution of 1.5 g. of N-(2,4-dinitro-6-trifluoromethylphenyl)-N-methyl-3-(1,1,2,2-tetrafluoroethoxy)benzenamine in 30 ml. of acetic acid containing 10 ml. of water were added in one portion 1.5 ml. of bromine. The reaction mixture was stirred for three hours at room temperature, and then was diluted by the addition of 30 ml. of watter. The mixture was stirred for an additional one hour, and then the solvent was decanted to leave a solid precipitate. The solid was dissolved in diethyl ether, dried, and the solvent was removed by evaporation under reduced pressure to afford 1.8 g. of N-(2,4-dinitro-6-trifluoromethylphenyl)-N-methyl-2,4-dibromo-5-(1,1,2,2-tetrafluoroethoxy)benzenamine.

M.P. 97—99°C.
Analysis calculated for $C_{16}H_8Br_2F_7N_3O_5$
Theory: C, 31.25; H, 1.31; N, 6.83
Found: C, 31.26; H, 1.38; N, 6.92

Examples 21—22

Following the procedure of Example 20, a halogen was reacted with a benzenamine to give the following halogenated benzenamines:

N-(2,4-dinitro-6-trifluoromethylphenyl)-2,6-dichloro-4-(1,1,2,2-tetrafluoroethoxy)benzenamine.

M.P. 80—82°C.
Analysis calculated for $C_{15}H_6Cl_2F_7N_3O_5$
Theory: C, 35.18; H, 1.18; N, 8.21
Found: C, 35.48; H, 1.31; N, 8.50

N-(2,4-dinitro-6-trifluoromethylphenyl)-2,6-dibromo-4-(1,1,2,2 -tetrafluoroethoxy)benzenamine.

M.P. 92—93°C.
Analysis calculated for $C_{15}H_6Br_2F_7N_3O_5$
Theory: C, 29.98; H, 1.01; N, 6.99
Found: C, 30.12; H, 1.05; N, 3.17

Example 23

N-(2,4,6-trinitrophenyl)-2,4-dibromo-5-(1,1,2,2-tetrafluoroethoxy)benzenamine.

To a stirred solution of 1.7 g. of N-(2,4,6-trinitrophenyl)-3-(1,1,2,2-tetrafluoroethoxy)-benzenamine in 20 ml. of dichloromethane were added in one portion of 1.5 ml. of bromine. The reaction mixture was heated at reflux for sixteen hours, and then 1.0 ml. of bromine was added. The mixture was refluxed for an additional two hours, and then cooled to room temperature. The precipitate which formed was collected by filtration to provide 1.4 g. of N-(2,4,6-trinitrophenyl)-2,4-dibromo-5-(1,1,2,2-tetrafluoroethoxy)benzenamine.

M.P. 201—203°C.
Analysis calculated for $C_{14}H_5Br_2F_4N_4O_7$
Theory: C, 29.09; H, 1.05; N, 9.69
Found: C, 29.35; H, 0.96; N, 9.81

Examples 24—26

Various benzenamines were halogenated in dichloromethane by the procedure of Example 23 to give the following halo substituted benzenamines:

N-(2,6-dinitro-4-trifluoromethylphenyl)-2,4-dichloro-5-(1,1,2,2-tetrafluoroethoxy)benzenamine.

M.P. 142—143°C.
Analysis calculated for $C_{15}H_6Cl_2F_7N_3O_5$
Theory: C, 35.18; H, 1.18; N, 8.21
Found: C, 35.48; H, 1.19; N, 8.12

N-(2,6-dinitro-4-trifluoromethylphenyl)-2,4-dibromo-5-(1,1,2,2-tetrafluoroethoxy)benzenamine.

M.P. 161—163°C.
Analysis calculated for $C_{15}H_6Br_2F_7N_3O_6$
Theory: C, 29.98; H, 1.01; N, 6.99
Found: C, 30.04; H, 1.18; N, 7.14

N-(2,4-dinitro-6-trifluoromethylphenyl)-2,4-dibromo-5-(1,1,2,2-tetrafluoroethoxy)benzenamine. (oil)

Analysis calculated for $C_{15}H_6Br_2F_7N_3O_5$
Theory: C, 36.94; H, 1.77; N, 9.24
Found: C, 37.24; H, 2.05; N, 9.55

As hereinbefore pointed out, the compounds of this invention have several utilities, including plant and soil antifungicidal activity. In a further embodiment of this invention, there are provided formulations for use in treating plant and soil fungicidal infections. Such formulations comprise a benzenamine of the above formula admixed with a suitable carrier, diluent or excipient therefor.

As used herein, the term "carrier", "diluent" or "excipient" refers to a material or materials with formulated to facilitate its storage, transport, handling, and administration to animals or application to plants or soil. Such carriers, diluents and excipients can be solid or liquid, and are preferbly substantially inert chemically and biologically. Commonly used solid carriers for use in dust formulations include gypsum, tripolite, diatomaceous earth, mineral silicates, vermiculite, talc, clays, calcium and magnesium limes, calcite and related can be employed if desired. Typical liquid carriers include water, saline, or organic fluids such as oils, for example a horticultural petroleum product.

The formulations comprehended by this invention comprise a suitable carrier, diluent or excipient admixed with a benzenamine of the above formula. The formulations will contain from about 5 to about 90 percent by weight of a benzenamine provided by this invention. A preferred concentration of active ingredient is from about 20 to about 80 percent by weight. The combination can take the form of concentrates, for instance aqueous emulsions or the like, or as sprays, dry powders, wettable powders, and the like. Preferred formulations include wettable powders, which are solid compositions of matter wherein the benzenamine is absorbed or absorbed in or on a sorptive carrier such as finely divided clay, talc, gypsum, lime, wood flour, kieserlguhr or the like. Wettable powder formulations commonly contain a small amount of a wetting, dispersing or emulsifying agent to facilitate dispersion in water or other liquid carriers utilized to distribute the active ingredient to the locus of desired fungus control. Commonly used wetting agents include condensed aryl sulfonic acids and sodium salts thereof, alkyl aryl polyether alcohols, sulfonated nonionic blends, anionic wetting agents, and the like.

Other preferred formulations according to this invention are emulsifiable concentrates, which are homogenous liquid or paste compositions of benzenamines which readily disperse in water or other liquid carrier to make a liquid which facilitates application to the locus of desired fungus control. Typical emulsifiable concentrates will contain a benzenamine of the above formula admixed with an organic solvent such as xylene, heavy aromatic naphthas, dioxane, or dimethylformamide, in addition to one or more emulsifying agents such as phenols, polyoxyethylene derivatives of sorbitan esters, complex ether-alcohols, and the like. Other ingredients such as antifreezes, antifoaming agents, stabilizers, antibaceterial agents and the like can be utilized if desired.

Example 27 and 28 which follow illustrate typical formulations contemplated herein.

Example 27
Wettable Powder Formulation

| Ingredient | | Percent by Weight |
|---|---|---|
| N-(2-trifluoromethyl-5-chloro-4,6-dinitrophenyl)-4-(1,1,2,2-tetrafluoroethoxy)benzenamine | | 25.8 |
| Stepanol ME | | 5.0 |
| Polyfon O | Wetting and dissipating agents | 5.0 |
| Zeolex-7 | | 10.0 |
| Bardens Clay | | 54.2 |

The above ingredients are mixed and air milled to provide a finely divided uniform powder. The powder is dispersed in water at the site of application and sprayed on the locus where fungal control is desired. The rate of application is about 5 to about 30 pounds of active ingredient per acre.

Example 28
Emulsifiable Concentrate Formulation

| Ingredient | Percent by Weight |
|---|---|
| N-(2,6-dinitro-4-trifluoromethylphenyl)-2,4-dichloro-5-(1,1,2,2-tetrafluoroethoxy benzenamine | 43.5 |
| Orthochlorotoluene | 18.5 |
| Ethoxylated polyoxypropylene glycol surfactant (atlox 8916TF) | 5.0 |
| Propylene glycol | 1.0 |
| Water | 33.0 |

The above ingredients are combined to form an aqueous emulsion which is to be diluted by additional water at the site of application. Such diluted formulation will be applied to the soil to be treated at a rate such that the active ingredient is about 10 to about 20 pounds per acre.

Example 29

The compounds provided by this invention have several utilities, including ectoparasitic activity, insecticidal activity, anticoccidial activity, as well as antifungal activity. A further embodiment of this invention is a method for treating and controlling fungal diseases in soil and on growing plants. The benzenamines defined by the above general formula can be applied as a drench to soil and incorporated therein prior to the emergence of plant seedlings, or can alternatively be applied to the foliage of growing plants such as cotton, beans, including soybeans and the like. The compounds are effective against a number of plant pathogenic fungi when applied per- or post-emergent at rates of about 0.1 to about 50 pounds per acre, idealy about 10 to about 20 pounds per acre. The compounds are effective in controlling and treating several common fungal diseases, including those caused by pathogens of the species Rhizoctonia, Fusarium (root rot), Verticillum (wilt), Pythium and the like.

A number of the compounds provided herein have been evaluated as antifungal agents in standard greenhouse tests. In a typical test, a benzenamine of this invention was formulated by mixing 70 mg. of the compound with 2 ml. of a solution comprising 500 ml. of ethanol, 500 ml. of acetone, and 100 ml. of Tween 20 (a polyoxyethylene sorbitan monolaurate made by Atlas Chemical Division of ICI America, Inc., Wilmington, Delaware). The mixture thus formed was diluted with 175 ml. of deionized water containing one drop of antifoam C emulsion per 2 liters of water. The final formulation contained 400 ppm. of benzenamine test compound, 10,000 ppm. of organic solvents, and 1,000 ppm.

of Tween 20. This solution was diluted with deionized water to obtain desired lower concentrations of active ingredient.

On the day a test was initiated, young expanding leaves of plants were detached and placed bottom side up in petri dishes containing filter paper placed on top of an expanding plastic mat to keep the leaves above the water flooding the bottom of the dishes. A water-soaked wad of cotton was wrapped around the petriole base of the leaf. The test chemical at the desired concentration was sprayed on the underside of the leaves to run off and the leaves were then allowed to dry. The dried leaves inoculated by spraying with a suspension of pathogenic fungi using a DeVilbiss sprayer. After inoculation, the dishes were placed in a moist chamber. The leaves were observed for disease symptoms and the results were recorded seven days after treatment. A rating scale of 1 to 5 was used to record the results, in which scale "1" equals severe disease, "2" equals moderately severe disease, "3" equals moderate disease, "4" equals slight disease and "5" equals no disease.

The results of such test is presented in Table I which follows. Column A in the table identifies the benzenamine evaluated. Column B gives the application rate in parts per million (ppm). Column C lists the rating of control against each of the indicated species of fungal infection.

TABLE I

| A<br>Compound Tested | B<br>Application Rate ppm | C<br>Disease Species and Control Rating | |
|---|---|---|---|
| N-(2,6-dinitro-4-trifluoro-methylphenyl)-N-methyl-3-(1,1,2,2-tetrafluoroethoxy)-benzenamine | 400 | Powdery Mildew<br>Rice Blast<br>Wheat Leaf Rust | 1<br>4<br>1 |
| N-(2,4-dinitro-6-trifluoro methylphenyl)-2,4-dibromo-5-(1,1,2,2-tetrafluoroethoxy)-benzenamine | 400 | Powdery Mildew<br>Rice Blast<br>Wheat Leaf Rust | 3<br>5<br>4 |
| N-(2-trifluoromethyl-4-nitro phenyl)-3-(1,1,2,2-tetrafluoro ethoxy)benzenamine | 400 | Powdery Mildew<br>Anthracnose<br>Rice Blast | 1<br>3<br>1 |
| N-(2,6-dinitro-4-trifluoromethylphenyl)-2,4-dichloro-5-(1,1,2,2-tetrafluoroethoxy)-benzenamine | 400 | Powdery Mildew<br>Rice Blast<br>Wheat Leaf Rust | 1<br>1<br>1 |

Compounds of this invention have additionally been evaluated in soil disease tests to demonstrate their antifungal activity. Test compounds were formulated by dissolving 57 mg. of compound in 1 ml. of a fifty percent (V/V) solution of acetone and ethanol. A 0.1% aqueous solution of Tween 20 was added to bring the final volume to 16 ml.

Pathogen-infested soil was placed in 8 oz. paper cups. A depression was made in the surface of the soil and 3 g. of Celatom MP-78 granules were placed in the depression. A 4 ml. aliquot of chemical formulation was added to the granules, and the cups were then covered with lids. The containers are shaken by hand for about 10 seconds, and then placed on a roller for about 10 minutes to thoroughly incorporate the test chemical into the soil. The treated soil was transferred to a 2.5 inch round plastic pot, and seeds of the host plant were added, and covered with additional treated soil. The effect of the test compounds was observed on the growing plants and was rated on a scale of 1 to 5 (1 is severe disease, 5 is no disease). The results of such evaluations are presented in Table II. Column A lists the compounds evaluated. Column B lists the application rates in pounds per acre (lbs/ac). Column C records the plant variety. Column D lists the pathogen and the disease rating for the tested compound.

TABLE II

| A<br>Compound Evaluated | B<br>Application Rate (lbs./A) | C<br>Plant Variety | D<br>Disease Rating | |
|---|---|---|---|---|
| N-(2,4-dinitro-6-trifluoromethylphenyl)-3-(1,1,2,2-tetrafluoroethoxy)benzenamine | 40 | Cotton | Rhizoctonia | 5 |
| | 40 | Cotton | Rhizoctonia | 4 |
| | 20 | Cotton | Rhizoctonia | 5 |
| | 10 | Cotton | Rhizoctonia | 5 |
| | 40 | Cotton | Pythium | 1 |
| | 40 | Beans | Fusarium | 1 |
| | 40 | Cotton | Verticillium | 1 |
| N-(2,4-dinitro-6-trifluoromethylphenyl)-2,4-dibromo-5-(1,1,2,2-tetrafluoroethoxy)benzenamine | 40 | Cotton | Rhizoctonia | 5 |
| | 40 | Cotton | Rhizoctonia | 5 |
| | 20 | Cotton | Rhizoctonia | 5 |
| | 10 | Cotton | Rhizoctonia | 5 |
| | 10 | Cotton | Rhizoctonia | 5 |
| | 5 | Cotton | Rhizoctonia | 3 |
| | 2.5 | Cotton | Rhizoctonia | 3 |
| | 40 | Beans | Fusarium | 1 |
| | 40 | Cotton | Verticillium | 1 |
| N-(2,6-dinitro-4-trifluoromethylphenyl)-2,4-dichloro-3-(1,1,2,2-tetrafluoroethoxy)benzenamine | 40 | Cotton | Rhizoctonia | 1 |
| | 40 | Cotton | Pythium | 1 |
| | 40 | Beans | Fusarium | 4 |
| | 40 | Beans | Fusarium | 3 |
| | 20 | Beans | Fusarium | 3 |
| | 10 | Beans | Fusarium | 3 |
| | 40 | Cotton | Verticillium | 1 |

TABLE II (continued)

| A<br>Compound Evaluated | B<br>Application Rate (lbs./A) | C<br>Plant Variety | D<br>Disease Rating | |
|---|---|---|---|---|
| N-(2-trifluoromethyl-4-nitrophenyl)-3-(1,1,2,2-tetrafluoroethoxy) benzenamine | 40 | Cotton | Rhizoctonia | 4 |
| | 40 | Cotton | Rhizoctonia | 4 |
| | 20 | Cotton | Rhizoctonia | 3 |
| | 10 | Cotton | Rhizoctonia | 3 |
| | 40 | Cotton | Pythium | 4 |
| | 40 | Cotton | Pythium | 3 |
| | 20 | Cotton | Pythium | 3 |
| | 10 | Cotton | Pythium | 1 |
| | 40 | Beans | Fusarium | 4 |
| | 40 | Beans | Fusarium | 4 |
| | 20 | Beans | Fusarium | 4 |
| | 10 | Beans | Fusarium | 4 |
| | 40 | Cotton | Verticillium | 1 |
| N-(2,4,6-trinitrophenyl)-2,4-dibromo-5-(1,1,2,2-tetrafluoroethoxy)benzen-amine | 40 | Cotton | Rhizoctonia | 1 |
| | 40 | Cotton | Pythium | 1 |
| | 40 | Cotton | Verticillium | 1 |
| | 40 | Beans | Fusarium | 1 |
| N-(2,4-dinitro-6-trifluoromethylphenyl)-N-methyl-3-(1,1,2,2-tetra-fluoroethoxy)benzenamine | 40 | Cotton | Rhizoctonia | 2 |
| | 40 | Cotton | Pythium | 3 |
| | 40 | Beans | Fusarium | 1 |
| | 40 | Cotton | Verticillium | 5 |
| | 40 | Cotton | Verticillium | 1 |
| | 20 | Cotton | Verticillium | 1 |
| | 10 | Cotton | Verticillium | 1 |

The data thus presented in Tables I and II demonstrate that the compounds of this invention can be used to control or treat diseases occurring in soil and plants and caused by pathogenic fungi. A further embodiment of this invention accordingly is an anti-fungal method which comprising applying to the locus to be treated an antifungal amount of a benzenamine of this invention. As already noted, the compounds can be formulated in any of a number of ways to facilitate convenient application to soil or to growing plants. The compounds are preferably applied as a liquid spray or drench, or alternatively as a dust or granule. While the specific rate of application of benzenamine may vary depending upon the disease to be treated, the host plant, the soil conditions of moisture and texture, the typical rate of application of benzenamine may vary depending upon the disease to be treated, the host plant, the soil conditions of moisture and texture, the typical rate of application for field use will be about 0.1 to about 50 pounds per acre, and more preferably about 10 to about 20 pounds per acre.

The benzenamines of this invention have also demonstrated good anticoccidal activity and good ectoparasitic activity. For example, N-(2,4-dinitro-6-trifluoromethylphenyl)-3-(1,1,2,2-tetrafluoroethoxy)benzenamine demonstrated complete control of adult housefly and blowfly larva in standard tests designed to show ectroparasitic activity. Compounds such as N-(2,4-dinitro-3-chloro-6-trifluoromethylphenyl)-4-(1,1,2,2-tetrafluoroethoxy)benzenamine and N-(2,4-dinitro-3-bromo-6-trifluoromethylphenyl)-4-(1,1,2,2,2-pentafluoroethoxy)benzenamine are contemplated as excellent insecticidal and anticoccidial agents.

Example 30

The benzenamines described above and defined by the above general formula are utilized according to this invention in the treatment and control of coccidiosis in animals. The method of this invention may be practiced for the prophylactic control of coccidiosis, for instance by the routine and continued administration to an animal susceptible to coccidiosis of an effective dose of a benzenamine, as well as for the therapeutic treatment of coccidiosis in animals so infected. The compounds can be formulated for convenient administration to animals by any of a number of routines, including the oral, intramuscular, introvenous, subcutaneous and related routes. The compounds are preferably formulated for systemic administration to animals such as bovine. As already noted, a preferred method according to the invention comprises treating poultry for coccidial infections. The benzenamines defined above are especially useful in treating and in aiding in the prevention of coccidiosis in poultry caused by *Eimeria necatrix, E. tenella, E. acervulina, E. brunetti, E. mivati,* and *E, maxima.* The method of this invention is ideally suited to the prevention of coccidiosis in broiler chickens.

For treatment of poultry according to this invention, the benzenamines are preferably formulated for oral administration, for instance as a feedstuff, by addition to the normal daily feed ration of the animals. Ideally, the benzenamine anticoccidial agent will be uniformly dispersed throughout a finished animal feed mixture. Such medicated feed mixture is then administered *ad lib:* to animals such as chickens and turkeys. The normal concentration of benzenamine to be employed in a feedstuff will be from about 20 grams per ton to about 500 grams per ton, and more preferably about 100 g/T to about 300 g/T. Poultry will routinely consume about 5 to about 250 grams of such feedstuff per day, depending upon size and age of the bird.

Any of a number of poultry feedstuffs can be utilized as a suitable carrier or diluent for the benzenamines defined above. Typical feedstuffs include the following:

Broiler Starter

| Ingredients | Percent |
|---|---|
| Corn, Yellow, Ground | 50.0 |
| Soybean Oil Meal, Solvent Extracted, Dehulled (50%) | 30.9 |
| Animal Fat | 6.5 |
| Fish Meal with Solubles (60%) | 5.0 |
| Corn Distillers Dried Solubles | 4.0 |
| Dicalcium Phosphate, Feed Grade | 1.8 |
| Calcium Carbonate (Ground Limestone) | 0.8 |
| Vitamin Premix TK-1 (1.03)*1/* | 0.5 |
| Salt (NaCl) | 0.3 |
| Trace Mineral Premix TK-01 (1.02) *2/* | 0.1 |
| Methionine Hydroxy Analog | 0.1 |
| Total | 100.0 |

Broiler Grower

| Ingredients | Percent |
|---|---|
| Corn, Yellow, Ground | 57.7 |
| Soybean Meal, Solvent, Extracted, Dehulled (50%) | 31.7 |
| Animal Fat (Beef tallow) | 6.0 |
| Dicalcium Phosphate, Feed Grade | 2.7 |
| Calcium Carbonate (Ground Limestone) | 0.9 |
| Vitamin Premix TK-01 (1.03 *1/* | 0.5 |
| Salt (NaCl) | 0.2 |
| Methionine Hydroxy Analog | 0.2 |
| Trace Mineral Premix TK-01 (1.02) *2/* | 0.1 |
| Total | 100.0 |

Chick Starter, Light Breeds

| Ingredients | Percent |
|---|---|
| Corn, Yellow, Ground | 56.3 |
| Soybean Meal, Solvent Extracted, Dehulled (50%) | 17.9 |
| Wheat Middlings | 10.0 |
| Corn Distillers Dried Solubles | 5.0 |
| Fish Meal with Solubles | 5.0 |
| Alfalfa Meal, Dehydrated (17%) | 2.5 |
| Dicalcium Phosphate, Feed Grade | 1.3 |
| Calcium Carbonate | 0.9 |
| Vitamin Premix[1] | 0.5 |
| Salt (NaCl) | 0.3 |
| Methionine Hydroxy Analog | 0.2 |
| Trace Mineral Premix[2] | 0.0 |
| Total | 100.0 |

Pullet Grower

| Ingredients | Percent |
|---|---|
| Corn, Yellow Ground | 73.5 |
| Soybean Meal, Solvent Extracted, Dehulled (50%) | 21.9 |
| Dicalcium Phosphate, Feed Grade | 2.5 |
| Calcium Carbonate | 1.0 |
| Vitamin Premix[1] | 0.5 |
| Salt (NaCl) | 0.3 |
| Methionine Hydroxy Analog | 0.2 |
| Trace Mineral Premix[2] | 0.1 |
| Total | 100.0 |

Pullet Developer

| Ingredients | Percent |
| --- | --- |
| Corn, Yellow, Ground | 67.5 |
| Oats, Ground Whole | 15.0 |
| Soybean Meal, Solvent Extracted, Dehulled (50%) | 13.4 |
| Dicalcium Phosphate, Feed Grade | 2.1 |
| Calcium Carbonate | 1.0 |
| Vitamin Premix[1] | 0.5 |
| Methionine Hydroxy Analog | 0.3 |
| Salt (NaCl) | 0.2 |
| Trace Mineral Premix[2] | 0.1 |
| Total | 100.0 |

Turkey Starter

| Ingredients | Percent |
| --- | --- |
| Soybean Meal, Solvent Extracted, Dehulled | 40.7 |
| Corn, Yellow, Ground | 39.7 |
| Fish Meal with Solubles | 5.0 |
| Beef Tallow | 5.0 |
| Corn Distillers Dried Solubles | 2.5 |
| Alfalfa Meal, Dehydrated (17%) | 2.5 |
| Dicalcium Phosphate, Feed Grade | 2.5 |
| Calcium Carbonate | 1.2 |
| Vitamin Premix[1] | 0.5 |
| Salt (NaCl) | 0.2 |
| Trace Mineral Premix[2] | 0.1 |
| Methionine Hydroxy Analog | 0.1 |
| Total | 100.0 |

Turkey Finisher

| Ingredients | Percent |
|---|---|
| Corn, Yellow, Ground | 71.2 |
| Soybean Meal, Solvent Extracted, Dehulled (50%) | 9.9 |
| Corn Distillers Dried Solbules | 5.0 |
| Alfalfa Meal, Dehydrated (17%) | 5.0 |
| Animal Fat | 3.0 |
| Fish Meal with Solubles | 2.5 |
| Dicalcium Phosphate, Feed Grade | 1.7 |
| Calcium Carbonate | 0.5 |
| Vitamin Premix[1] | 0.5 |
| Salt (NaCl) | 0.4 |
| Methionine Hydroxy Analog | 0.2 |
| Trace Mineral Premix[2] | 0.1 |
| Total | 100.0 |

1/ Vitamin premix provides 3000 IU of vitamin A, 900 ICU of vitamin D, 40 mg. of vitamin E. 0.7 mg. of vitamin K, 1000 mg. of choline, 70 mg. of niacin, 4 mg. of pantothenic acid, 4 mg. of riboflavin, 0.10 mg. of vitamin $B_{12}$, 0.10 mg. of biotin and 125 mg. of ethoxyquin per kg. of complete feed.
2/ Trace mineral premix provides 75 mg. of manganese, 50 mg. of zinc, 25 mg. of iron and 1 mg of iodine per kg. of complete feed.

A benzenamine anticoccidial agent can be admixed with any such poultry feedstuffs so that the final feedstuff contains about 20 to about 500 grams of benzenamine per ton of feedstuff. For example, about 300 g. of a compound such as N-(2,4-dinitro-6-trifluoromethylphenyl)-4-(trifluoromethoxy)-benzenamine can be added to about one ton of the above-noted Broiler Grower mixture for use according to this invention. Similarly, about 200 to about 300 g. of N-(2-trifluoromethyl-5-chloro-4,6-dinitrophenyl)-4-(1,1,2,2-tetrafluoroethoxy)benzenamine can be admixed to uniformity with about one ton of the above-described Turkey Finisher for administration to turkeys pursuant to the method of this invention.

The benzenamines defined above can alternatively be formulated as a feedstuff pre-mix. For example, a benzenamine can be admixed with a substantially inert carrier such as ground rice hulls, soybean meal, wheat middlings, fermentation mycelia and the like. Nutritive carriers such as cereals can also be utilized. Such mixture of carrier and benzenamine anticoccidiostat will preferably contain about 5 to about 90 percent by weight of the benzenamine anticoccidial agent, and more preferably about 20 to about 70.percent by weight. Such pre-mix formulation is then mixed with a normal feed ration at a rate so that the active ingredient is present in about 20 to about 500 grams per ton of final feed ration.

Still another formulation which can be utilized according to this invention comprises a benzenamine of the above formula substantially disolved in drinking water, for example in the drinking water of poultry such as chickens and turkeys. For such formulations, it is occasionally preferred to utilize a physiologically-acceptable salt such as the sodium or potassium salt, which compounds generally are substantially water soluble. For such formulation, it is often convenient to prepare watersoluble powders or dispersible powders comprising a benzenamine admixed with carriers such as dextrose, sucrose, dimethyl sulfoxide, or other suitable diluent. Typically, the benzenamine with be present in such forms in about 0.01 to about 30 percent by weight. Such powder or liquid formulations are conveniently added to the poultry drinking water at the side of administration.

The anticoccidial activity of the N-(nitrophenyl)polyfluoroalkoxybenzenamines defined by the above formula has been determined in standard *in vivo* tests in chickens. A typical evaluation was conducted as follows:

one-week-old broiler chicks were alloted to five-bird cages and were fed a medicated or control ration, typically for one day, prior to infection with oocysts of a coccidiosis-causing organism. The chicks were maintained on their respective rations for a period of time, typically seven days. Generally, there were from three to six replicates per treatment. Anticoccidial efficacy was typically determined by the lesion scores, but other measures of efficacy were employed in many of the tests. In determining lesion scores, the birds were sacrificed and the severity of lesions scored on a 0—4 scale, with lession-free birds scored as 0, extremely severe infections scored as 4, and intermediate degrees of infections scored as 1, 2, or 3. The scores of all birds which received as given treatment were averaged.

Table III below presents the results for the evaluation of N-(2,4-dinitro-6-trifluoromethylphenyl)-4-(1,1,2,2-tetrafluoroethoxy)benzenamine. The results are a statistical analysis of intestinal lesion scores of broiler cockerels utilizing the Duncans Multiple Range Test ($P<0.05$) and Gebhardts algorithm for unequal sample size. The data is reported with superscript letters, and those data not followed by a common letter are significantly different. The animals were inoculated with strains of *Eimeria acervulina* (strain 59) and *Eimeria maxima* (strain F.S. 177)

TABLE III

| Treatment | Dose (ppm) | Intestinal Lesion Scores | |
|---|---|---|---|
| | | Replicates | Means |
| Infected controls (no medication) | | 3 | $4.93^{c}$ |
| N-(2,4-dinitro-6-trifluoromethylphenyl)-4-(1,1,2,2-tetrafluoroethoxy)benzenamine | 10 | 3 | $3.00^{b}$ |
| | 20 | 3 | $0.20^{a}$ |
| | 30 | 3 | $0.00^{a}$ |
| | 50 | 3 | $0.00^{a}$ |

A similar evaluation was carried out to compare a benzenamine of the invention with the anticoccidial agent known generally as monensin. Intestinal and cecal lesion scores were assigned to broiler cockerels inoculated with *Eimeria acervulina* (strain 59), *Eimeria tenella* (strain 155) and *Eimeria maxima* (strain F. S. 177). The results are presented in Table IV.

TABLE IV

| Treatment | Dose (ppm) | Lesion Scores | | | |
|---|---|---|---|---|---|
| | | Intestinal | | Cecal | |
| | | Replicates | Mean | Replicates | Mean |
| Infected control (no treatment) | | 2 | 5.60 | 2 | 3.55 |
| Monensin | 25 | 2 | 4.50 | 2 | 3.50 |
| | 50 | 2 | 0.90 | 2 | 1.73 |
| | 100 | 3 | 0.00 | 3 | 0.00 |
| N-(2,4-dinitro-6-trifluoromethylphenyl)-4-(1,1,2,2-tetrafluoroethoxy)benzenamine | 8 | 2 | 6.00 | 2 | 3.75 |
| | 15 | 2 | 1.87 | 2 | 3.30 |
| | 30 | 3 | 0.20 | 3 | 1.73 |

**Claims for the Contracting States: BE CH DE FR GB IT LI LU NL SE**

1. A compound of the formula

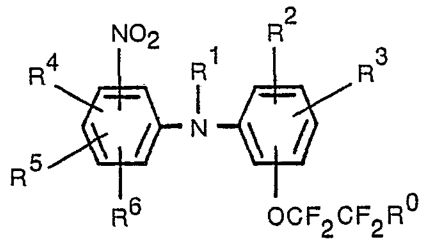

I

wherein:
$R^0$ is hydrogen or fluoro;
$R^1$ is hydrogen or $C_1$—$C_2$ alkyl;
$R^2$ and $R^3$ independently are hydrogen or halo;
$R^4$ is hydrogen, trifluoromethyl, cyano, $C_1$—$C_4$ alkyl, hydroxycarbonyl or $C_1$—$C_4$ alkoxycarbonyl;
$R^5$ is hydrogen, halo, nitro, hydroxy, methoxy or amino;
$R^6$ is hydrogen or nitro, and the physiologically-acceptable salts thereof.

2. The compound of Claim 1 which has the formula

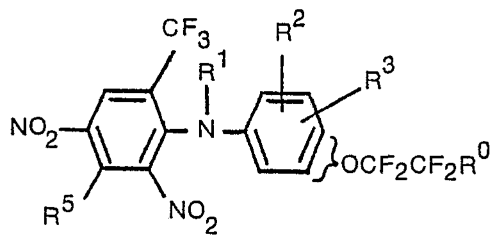

II

wherein:
$R^0$ is hydrogen or fluoro;
$R^1$ is hydrogen or $C_1$—$C_2$ alkyl;
$R^2$ and $R^3$ independently are hydrogen or halo;
$R^5$ is hydrogen or halo; and the physiologically-acceptable salts thereof.

3. The compound of Claim 1, wherein the compound is N-(2,4-dinitro-6-trifluoromethylphenyl)-2,4-dibromo-5-(1,1,2,2-tetrafluoroethoxy)benzenamine.

4. The compound of Claim 1, wherein the compound is N-(2,6-dinitro-4-trifluoromethylphenyl)-2,4-dichloro-5-(1,1,2,2-tetrafluoroethoxy)benzenamine.

5. The compound of Claim 1, wherein the compound is N-(2,4-dinitro-6-trifluoromethylphenyl)-4-(1,1,2,2-tetrafluoroethoxy)benzenamine.

6. A process for the preparation of a compound of the formula

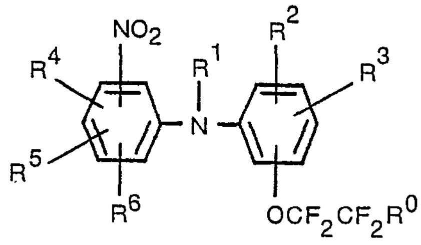

I

wherein:
$R^0$ is hydrogen or fluoro;
$R^1$ is hydrogen or $C_1$—$C_2$ alkyl;
$R^2$ and $R^3$ independently are hydrogen or halo;
$R^4$ is hydrogen, trifluoromethyl, cyano, $C_1$—$C_4$ alkyl, hydroxycarbonyl or $C_1$—$C_4$ alkoxycarbonyl;
$R^5$ is hydrogen, halo, nitro, hydroxy, methoxy or amino;
$R^6$ is hydrogen or nitro; and the physiologically-acceptable salts thereof, which comprises reacting in an unreactive organic solvent, in the presence of a strong base, about an equimolar quantity of a substituted phenyl electrophilic agent with a phenylamine derivative.

7. A process of Claim 6 wherein a polyfluoroethoxyphenyl electrophilic agent such as a phenyl halide is reacted with a nitrophenylamine according to the following scheme:

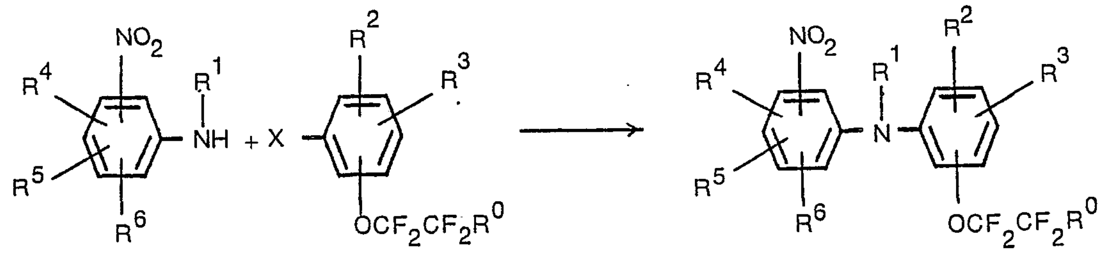

wherein $R^0$, $R^1$, $R^2$, $R^3$, $R^4$, $R^5$ and $R^6$ are as defined in Claim 6, and X is a good leaving group such as halo.

8. A process of Claim 6 wherein a nitrophenyl electrophilic agent is reacted with a polyfluoroethoxyphenylamine according to the following scheme:

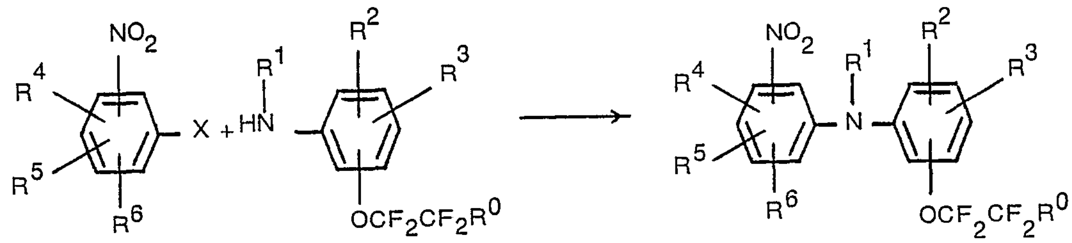

wherein $R^0$, $R^1$, $R^2$, $R^3$, $R^4$, $R^5$ and $R^6$ are as defined in Claim 6 above, and X is a good leaving group such as halo.

9. A process of Claim 6 wherein the substituted phenyl electrophilic agent is tetra or pentafluoroethoxyphenyl chloride.

10. The process of anyone of Claims 6 to 9 for the preparation of a compound which has the formula

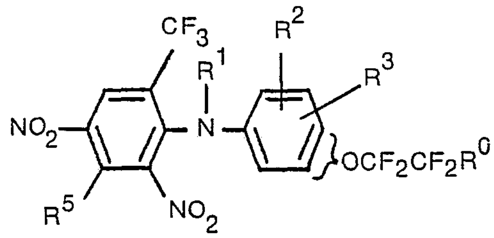

II

wherein:

$R^0$ is hydrogen or fluoro;

$R^1$ is hydrogen or $C_1$—$C_2$ alkyl;

$R^2$ and $R^3$ independently are hydrogen or halo;

$R^5$ is hydrogen or halo; and the physiologically-acceptable salts thereof.

11. A process for the preparation of a compound of Formula I wherein $R^1$ is $C_1$—$C_2$ alkyl which comprises alkylating a compound of Formula I wherein $R^1$ is hydrogen.

12. A process for the preparation of a compound of Formula I wherein $R^4$ is $C_1$—$C_4$ alkoxycarbonyl comprises esterifying a compound of Formula I wherein $R^4$ is hydroxycarbonyl.

13. A process for the preparation of a compound of Formula I wherein $R^2$ and $R^3$ are independently halo which comprises halogenating a compound Formula I wherein $R^2$ and $R^3$ are hydrogen.

14. A process for the preparation of the physiologically acceptable salts of Formula I which comprises reacting a compound of Formula I wherein $R^1$ is hydrogen with a base.

15. The process of Claim 6, wherein the compound prepared is as defined in any of claims 3, 4 and 5.

16. An anticoccidial formulation which comprises an anticoccidial benzenamine of the formula

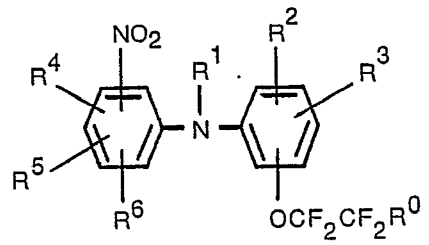

I

wherein:

$R^0$ is hydrogen or fluoro;

$R^1$ is hydrogen or $C_1$—$C_2$ alkyl;

$R^2$ and $R^3$ independently are hydrogen or halo;

$R^4$ is hydrogen, trifluoromethyl, cyano, $C_1$—$C_4$ alkyl, hydroxycarbonyl, or $C_1$—$C_4$ alkoxycarbonyl;

$R^5$ is hydrogen, halo, nitro, hydroxy, methoxy or amino;

$R^6$ is hydrogen or nitro; and the physiologically-acceptable salts thereof; together with a suitable carrier thereof.

17. The formulation of Claim 16 wherein the active ingredient has the formula

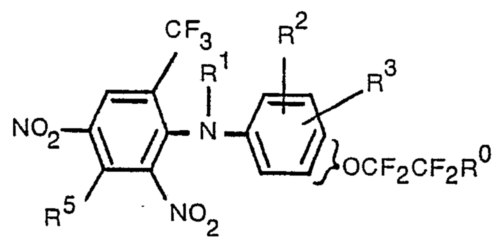

II

wherein:

$R^0$ is hydrogen or fluoro;

$R^1$ is hydrogen or $C_1$—$C_2$ alkyl;

$R^2$ and $R^3$ independently are hydrogen or halo;

$R^5$ is hydrogen or halo; and the physiologically-acceptable salts thereof.

18. The formulation of Claim 16 or 17 wherein the active ingredient is N-(2,4-dinitro-6-trifluoromethylphenyl)-4-(1,1,2,2-tetrafluoroethoxy)benzenamine.

19. The formulation of Claim 16, 17 or 18 wherein the combination is a poultry feedstuff.

20. The formulation of Claim 16, 17 or 18 wherein the combination is a poultry pre-mix.

21. A formulation useful in the treatment of fungal infections in soil and on plants comprising an antifungal amount of a compound of the formula

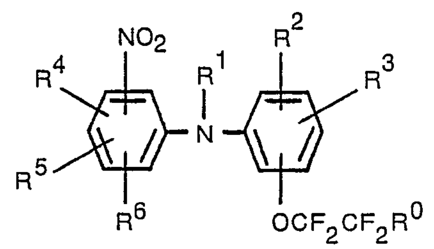

I

wherein:

$R^0$ is hydrogen or fluoro;

$R^1$ is hydrogen or $C_1$—$C_2$ alkyl;

$R^2$ and $R^3$ independently are hydrogen or halo;

$R^4$ is hydrogen, trifluoromethyl, cyano, $C_1$—$C_4$ alkyl, hydroxycarbonyl or $C_1$—$C_4$ alkoxycarbonyl;

$R^5$ is hydrogen, halo, nitro, hydroxy, methoxy or amino;

$R^6$ is hydrogen or nitro; and the physiologically-acceptable salts thereof, and a suitable carrier therefor.

22. The formulation of Claim 21 wherein the active ingredient is as defined in any of Claims 2, 3 and 4.

23. The formulation of Claim 21 wherein the active ingredient is N-(2,4-dinitro-6-trifluoromethylphenyl)-3-(1,1,2,2-tetrafluoroethoxy)benzenamine.

24. The formulation of Claim 21 wherein the active ingredient is N-(2-trifluoromethyl-4-nitrophenyl)-3-(1,1,2,2-tetrafluoroethoxy)benzenamine.

25. A method for the treatment of plant and soil fungal infections which comprises applying to the locus to be treated an antifungal amount of a compound of the formula

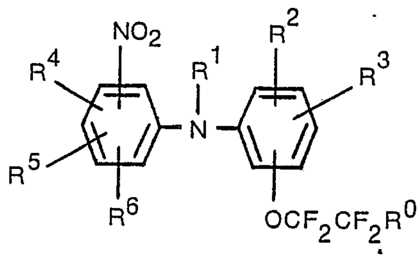

I

wherein:

$R^0$ is hydrogen or fluoro;

$R^1$ is hydrogen or $C_1$—$C_2$ alkyl;

$R^2$ and $R^3$ independently are hydrogen or halo;

$R^4$ is hydrogen, trifluoromethyl, cyano, $C_1$—$C_4$ alkyl, hydroxycarbonyl or $C_1$—$C_4$ alkoxycarbonyl;

$R^5$ is hydrogen, halo, nitro, hydroxy, methoxy or amino;

$R^6$ is hydrogen or nitro; and the physiologically-acceptable salts thereof.

26. The method of Claim 25 wherein the active compound applied is as defined in any of Claims 2, 3 and 4.

27. The method of Claim 25 wherein the active compound applied is N-(2,4-dinitro-6-trifluoromethylphenyl)-3-(1,1,2,2-tetrafluoroethoxy)benzenamine.

28. The method of Claim 25 wherein the active compound applied is N-(2-trifluoromethyl-4-nitrophenyl)-3-(1,1,2,2-tetrafluoroethoxy)benzenamine.

29. An insecticidal or ectoparisiticidal composition comprising a compound according to Claim 1 associated with a suitable carrier therefor.

**Claims for the Contracting State: AT**

1. A process for the preparation of a compound of the formula

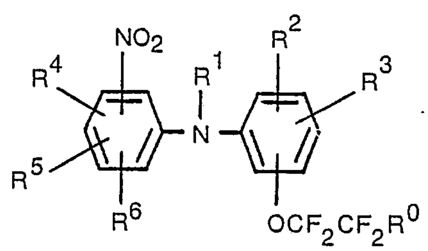

I

wherein:

$R^0$ is hydrogen or fluoro;

$R^1$ is hydrogen or $C_1$—$C_2$ alkyl;

$R^2$ and $R^3$ independently are hydrogen or halo;

$R^4$ is hydrogen, trifluoromethyl, cyano, $C_1$—$C_4$ alkyl, hydroxycarbonyl or $C_1$—$C_4$ alkoxycarbonyl;

$R^5$ is hydrogen, halo, nitro, hydroxy, methoxy or amino;

$R^6$ is hydrogen or nitro; and the physiologically-acceptable salts thereof, which comprises reacting in an unreactive organic solvent, in the presence of a strong base, about an equimolar quantity of a substituted phenyl electrophilic agent with a phenylamine derivative.

2. A process of Claim 1 wherein a polyfluoroethoxyphenyl electrophilic agent such as a phenyl halide is reacted with a nitrophenylamine according to the following scheme:

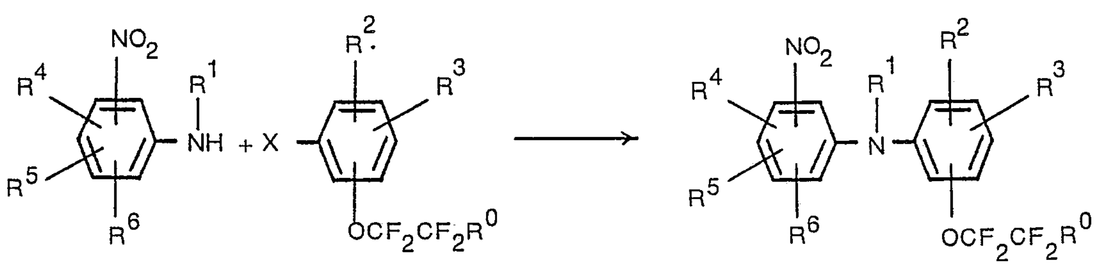

wherein $R^0$, $R^1$, $R^2$, $R^3$, $R^4$, $R^5$ and $R^6$ are as defined in Claim 6, and X is a good leaving group such as halo.

3. A process of Claim 1 wherein a nitrophenyl electrophilic agent is reacted with a polyfluoroethoxyphenylamine according to the following scheme:

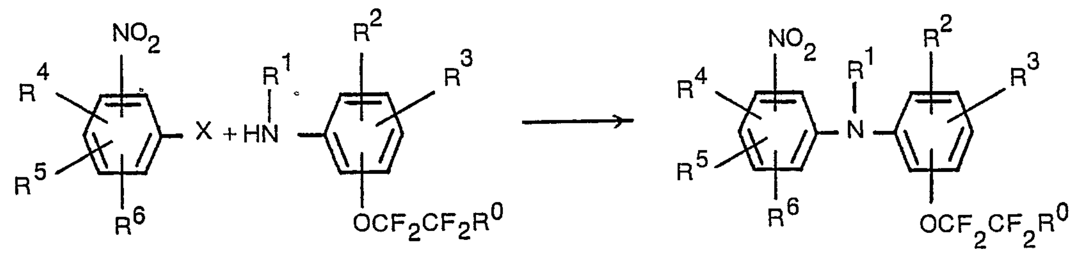

wherein $R^0$, $R^1$, $R^2$, $R^3$, $R^4$, $R^5$ and $R^6$ are as defined in Claim 6 above, and X is a good leaving group such as halo.

4. A process of Claim 1 wherein the substituted phenyl electrophilic agent is tetra or pentafluoroethoxyphenyl chloride.

5. The process of anyone of Claims 1 to 4 for the preparation of a compound which has the formula

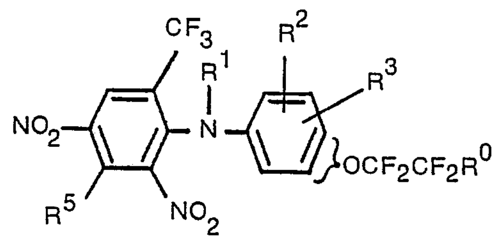

II

wherein:

$R^0$ is hydrogen or fluoro;

$R^1$ is hydrogen or $C_1$—$C_2$ alkyl;

$R^2$ and $R^3$ independently are hydrogen or halo;

$R^5$ is hydrogen or halo; and the physiologically-acceptable salts thereof.

6. A process for the preparation of a compound of Formula I wherein $R^1$ is $C_1$—$C_2$ alkyl which comprises alkylating a compound of Formula I wherein $R^1$ is hydrogen.

7. A process for the preparation of a compound of Formula I wherein $R^4$ is $C_1$—$C_4$ alkoxycarbonyl which comprises esterifying a compound of Formula I wherein $R^4$ is hydroxycarbonyl.

8. A process for the preparation of a compound of Formula I wherein $R^2$ and $R^3$ are independently halo which comprises halogenating a compound of Formula I wherein $R^2$ and $R^3$ are hydrogen.

9. A process for the preparation of the physiologically acceptable salts of Formula I which comprises reacting a compound of Formula I wherein $R^1$ is hydrogen with a base.

10. The process of Claim 1, wherein the compound prepared is N-(2,4-dinitro-6-trifluoromethylphenyl)-2,4-dibromo-5-(1,1,2,2-tetrafluoroethoxy)benzenamine.

11. The process of Claim 1, wherein the compound prepared is N-(2,6-dinitro-4-trifluoromethylphenyl)-2,4-dichloro-5-(1,1,2,2-tetrafluoroethoxy)benzenamine.

12. The process of Claim 1, wherein the compound prepared is N-(2,4-dinitro-6-trifluoromethylphenyl)-4-(1,1,2,2-tetrafluoroethoxy)benzenamine.

13. A formulation useful in the treatment of fungal infections in soil and on plants comprising an antifungal amount of a compound as defined in any of Claims 1, 5, 10, 11 or a physiologically-acceptable salt thereof, and a suitable carrier therefor.

14. The formulation of Claim 13 wherein the active ingredient is N-(2,4-dinitro-6-trifluoromethylphenyl)-3-(1,1,2,2-tetrafluoroethoxy)benzenamine.

15. The formulation of Claim 13 wherein the active ingredient is N-(2-trifluoromethyl-4-nitrophenyl)-3-(1,1,2,2-tetrafluoroethoxy)benzenamine.

16. A method for the treatment of plant and soil fungal infections which comprises applying to the locus to be treated an antifungal amount of a compound as defined in any of Claims 1, 5, 10, 11, 14 and 15, or a physiologically acceptable salt thereof.

17. An anticoccidial formulation which comprises an anticoccudial benzenamine as defined in Claim 1 and a suitable carrier therefor.

18. An insecticidal or ectoparisiticidal composition comprising a compound as defined in Claim 1 and a suitable carrier therefor.

**Revendications pour les Etats contractants: BE CH DE FR GB IT LI LU NL SE**

1. Composé de formule:

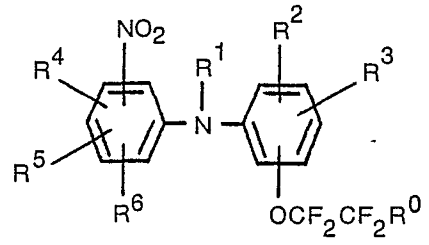

I

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^4$ représente un atome d'hydrogène, un groupe trifluorométhyle, un groupe cyano, un groupe alkyle en $C_1$—$C_4$, un groupe hydroxycarbonyle ou un groupe alcoxy(en $C_1$—$C_4$)carbonyle;

$R^5$ représente un atome d'hydrogène, un atome d'halogène, un groupe nitro, un groupe hydroxy, un groupe méthoxy ou un groupe amino;

$R^6$ représente un atome d'hydrogène ou un groupe nitro;

de même que les sels physiologiquement acceptables de ce composé.

2. Composé suivant la revendication 1, caractérisé en ce qu'il répond à la formule:

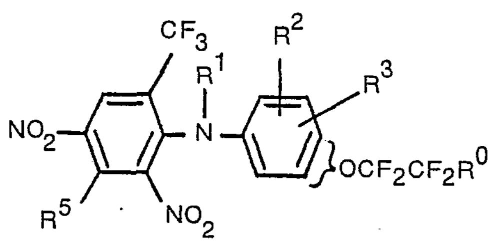

II

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^5$ représente un atome d'hydrogène ou un atome d'halogène;

de même que les sels physiologiquement acceptables de ce composé.

3. Composé suivant la revendication 1, caractérisé en ce qu'il est la N-(2,4-dinitro-6-trifluorométhylphényl)-2,4-dibromo-5-(1,1,2,2-tétrafluoréthoxy)benzénamine.

4. Composé suivant la revendication 1, caractérisé en ce qu'il est la N-(2,6-dinitro-4-trifluorométhylphényl) -2,4-dichloro-5-(1,1,2,2-tétrafluoréthoxy)benzénamine.

5. Composé suivant la revendication 1, caractérisé en ce qu'il est la N-(2,4-dinitro-6-trifluorométhylphényl)-4-(1,1,2,2-tétrafluoréthoxy)benzénamine.

6. Procédé de préparation d'un composé de formule:

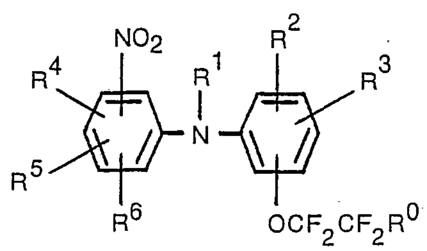

I

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^4$ représente un atome d'hydrogène, un groupe trifluorométhyle, un groupe cyano, un groupe alkyle en $C_1$—$C_4$, un groupe hydroxycarbonyle ou un groupe alcoxy(en $C_1$—$C_4$)carbonyle;

$R^5$ représente un atome d'hydrogène, un atome d'halogène, un groupe nitro, un groupe hydroxy, un groupe méthoxy ou un groupe amino;

$R^6$ représente un atome d'hydrogène ou un groupe nitro;

ainsi que des sels physiologiquement acceptables de ce composé, caractérisé en ce qu'il consiste à faire réagir, dans un solvant organique non réactif et en présence d'une base forte, une quantité à peu près équimolaire d'un agent électrophile phénylique substitué avec un dérivé de phénylamine.

7. Procédé suivant la revendication 6, caractérisé en ce qu'on fait réagir un agent électrophile polyfluoréthoxyphénylique tel qu'un halogénure de phényle avec une nitrophénylamine conformément au schéma suivant:

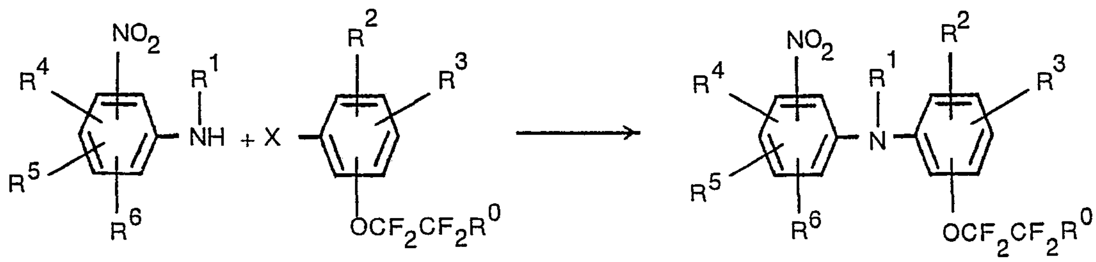

où $R^0$, $R^1$, $R^2$, $R^3$, $R^4$, $R^5$ et $R^6$ ont les significations définies dans la revendication 6 et X est un bon groupe qui s'éloigne tel qu'un atome d'halogène.

8. Procédé suivant la revendication 6, caractérisé en ce qu'on fait réagir un agent électrophile nitrophénylique avec une polyfluoréthoxyphénylamine conformément au schéma suivant:

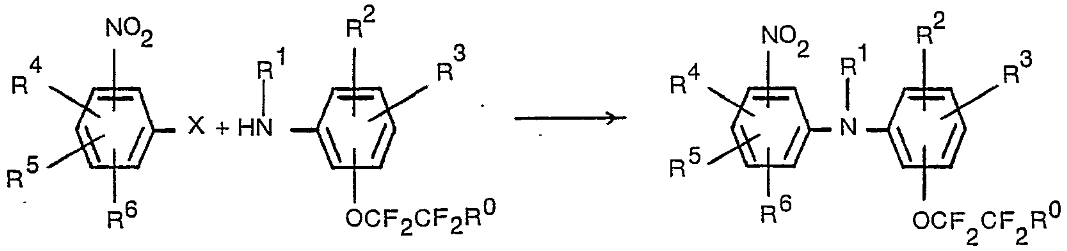

où $R^0$, $R^1$, $R^2$, $R^3$, $R^4$, $R^5$ et $R^6$ one les significations définies dans la revendication 6 ci-dessus et X est un bon groupe qui s'éloigne tel qu'un atome d'halogène.

9. Procédé suivant la revendication 6, caractérisé en ce que l'agent électrophile phénylique substitué est un chlorure de tétra- ou penta-fluoréthoxyphényle.

10. Procédé suivant l'une quelconque des revendications 6 à 9 pour la préparation d'un composé répondant à la formule:

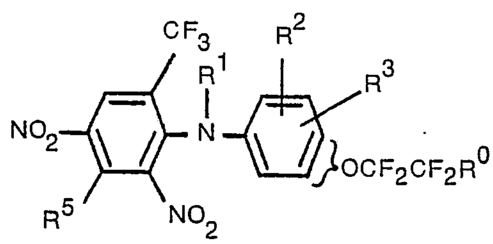

II

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^5$ représente un atome d'hydrogène ou un atome d'halogène;

ainsi que des sels physiologiquement acceptables de ce composé.

11. Procédé de préparation d'un composé de formule I dans laquelle $R^1$ représente un groupe alkyle en $C_1$—$C_2$, caractérisé en ce qu'il consiste à alkyler un composé de formule I dans laquelle $R^1$ représente un atome d'hydrogène.

12. Procédé de préparation d'un composé de formule I dans laquelle $R^4$ représente un groupe alcoxy(en $C_1$—$C_4$)carbonyle, caractérisé en ce qu'il consiste à estérifier un composé de formule I dans laquelle $R^4$ représente un groupe hydroxycarbonyle.

13. Procédé de préparation d'un composé de formule I dans laquelle $R^2$ et $R^3$ représentent chacun indépendamment l'un de l'autre un atome d'halogène, caractérisé en ce qu'il consiste à halogéner un composé de formule I dans laquelle $R^2$ et $R^3$ représentent chacun un atome d'hydrogène.

14. Procédé de préparation des sels physiologiquement acceptables de formule I, caractérisé en ce qu'il consiste à faire réagir un composé de formule I dans laquelle $R^1$ représente un atome d'hydrogène, avec une base.

15. Procédé suivant la revendication 6, caractérisé en ce que le composé préparé a la définition donnée dans l'une quelconque des revendications 3, 4, et 5.

16. Formulation anticoccidienne, caractérisée en ce qu'elle comprend une benzénamine anticoccidienne de formule:

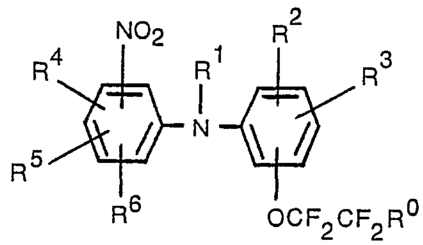 I

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^4$ représente un atome d'hydrogène, un groupe trifluorométhyle, un groupe cyano, un groupe alkyle en $C_1$—$C_4$, un groupe hydroxycarbonyle ou un groupe alcoxy(en $C_1$—$C_4$)carbonyle;

$R^5$ représente un atome d'hydrogène, un atome d'halogène, un groupe nitro, un groupe hydroxy, un groupe méthoxy ou un groupe amino;

$R^6$ représente un atome d'hydrogène ou un groupe nitro;

de même que les sels physiologiquement acceptables de cette benzénamine, conjointement avec un support approprié pour cette benzénamine et ses sels.

17. Formulation suivant la revendication 16, caractérisée en ce que l'ingrédient actif répond à la formule:

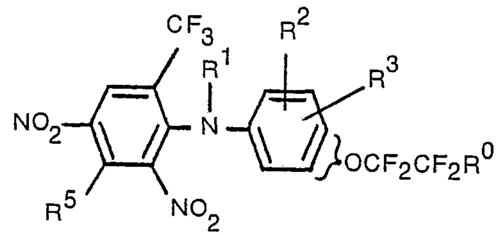 II

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^5$ représente un atome d'hydrogène ou un atome d'halogène;

de même que les sels physiologiquement acceptables de ce composé.

18. Formulation suivant la revendication 16 ou 17, caractérisée en ce que l'ingrédient actif est la N-(2,4-dinitro-6-trifluorométhylphényl)-4-(1,1,2,2-tétrafluoréthoxy)benzénamine.

19. Formulation suivant la revendication 16, 17 ou 18, caractérisée en ce que la combinaison est un aliment pour la volaille.

20. Formulation suivant la revendication 16, 17 ou 18, caractérisée en ce que la combinaison est un prémélange pour la volaille.

21. Formulation utile dans le traitement des infections fongiques du sol et des plantes, caractérisée en ce qu'elle comprend une quantité antifongique d'un composé de formule:

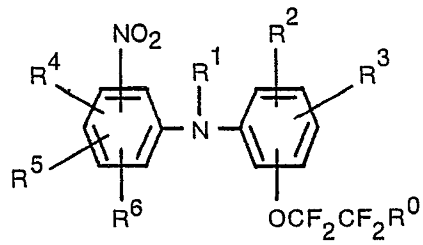

I

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^4$ représente un atome d'hydrogène, un groupe trifluorométhyle, un groupe cyano, un groupe alkyle en $C_1$—$C_4$, un groupe hydroxycarbonyle ou un groupe alcoxy(en $C_1$—$C_4$)carbonyle;

$R^5$ représente un atome d'hydrogène, un atome d'halogène, un groupe nitro, un groupe hydroxy, un groupe méthoxy ou un groupe amino;

$R^6$ représente un atome d'hydrogène ou un groupe nitro;

de même que les sels physiologiquement acceptables de ce composé et un support approprié pour ce composé ou ses sels.

22. Formulation suivant la revendication 21, caractérisée en ce que l'ingrédient actif a la définition donnée dans l'une quelconque des revendications 2, 3 et 4.

23. Formulation suivant la revendication 21, caractérisée en ce que l'ingrédient actif est la N-(2,4-dinitro-6-trifluorométhylphényl)-3-(1,1,2,2-tétrafluoroéthoxy)benzénamine.

24. Formulation suivant la revendication 21, caractérisée en ce que l'ingrédient actif est la N-(2-trifluorométhyl-4-nitrophényl)-3-(1,1,2,2-tétrafluoroéthoxy)benzénamine.

25. Procédé pour le traitement des infections fongiques des plantes et du sol, caractérisé en ce qu'il consiste à appliquer, à l'endroit à traiter, une quantité antifongique d'un composé de formule:

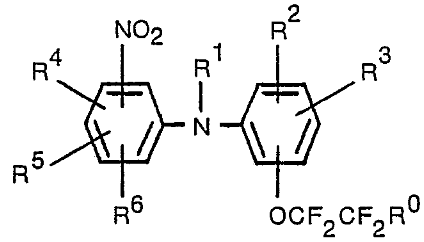

I

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^4$ représente un atome d'hydrogène, un groupe trifluorométhyle, un groupe cyano, un groupe alkyle en $C_1$—$C_4$, un groupe hydroxycarbonyle ou un groupe alcoxy(en $C_1$—$C_4$)carbonyle;

$R^5$ représente un atome d'hydrogène, un atome d'halogène, un groupe nitro, un groupe hydroxy, un groupe méthoxy ou un groupe amino;

$R^6$ représente un atome d'hydrogène ou un groupe nitro;

ainsi que des sels physiologiquement acceptables de ce composé.

26. Procédé suivant la revendication 25, caractérisé en ce que le composé actif appliqué a la définition donnée dans l'une quelconque des revendications 2, 3 et 4.

27. Procédé suivant la revendication 25, caractérisé en ce que le composé actif appliqué est la N-(2,4-dinitro-6-trifluorométhylphényl)-3-(1,1,2,2-tétrafluoroéthoxy)benzénamine.

28. Procédé suivant la revendication 25, caractérisé en ce que le composé actif appliqué est la N-(2-trifluorométhyl-4-nitrophényl)-3-(1,1,2,2-tétrafluoréthoxy)benzénamine.

29. Composition insecticide ou ectoparasiticide comprenant un composé suivant la revendication 1 en association avec un support approprié pour ce composé.

**Revendications pour l'Etat contractant: AT**

1. Procédé de préparation d'un composé de formule:

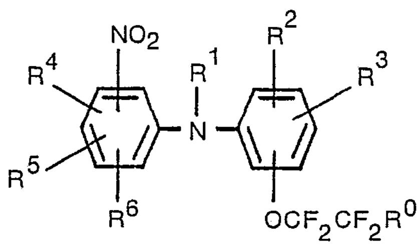

I

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^4$ représente un atome d'hydrogène, un groupe trifluorométhyle, un groupe cyano, un groupe alkyle en $C_1$—$C_4$, un groupe hydroxycarbonyle ou un groupe alcoxy(en $C_1$—$C_4$)carbonyle;

$R^5$ représente un atome d'hydrogène, un atome d'halogène, un groupe nitro, un groupe hydroxy, un groupe méthoxy ou un groupe amino;

$R^6$ représente un atome d'hydrogène ou un groupe nitro;

ainsi que des sels physiologiquement acceptables de ce composé, caractérisé en ce qu'il consiste à faire réagir, dans un solvant organique non réactif et en présence d'une base forte, une quantité à peu près équimolaire d'un agent électrophile phénylique substitué avec un dérivé de phénylamine.

2. Procédé suivant la revendication 1, caractérisé en ce qu'on fait réagir un agent électrophile polyfluoréthoxyphénylique tel qu'un halogénure de phényle avec une nitrophénylamine conformément au schéma suivant:

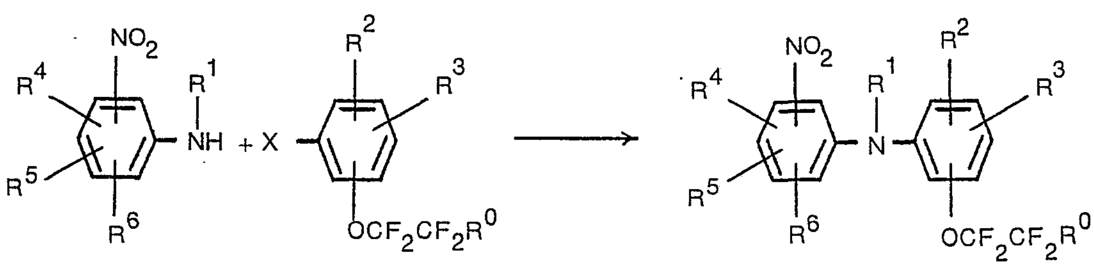

où $R^0$, $R^1$, $R^2$, $R^3$, $R^4$, $R^5$ et $R^6$ ont les significations définies dans la revendication 6, tandis que X est un bon groupe qui s'éloigne tel qu'un atome d'halogène.

3. Procédé suivant la revendication 1, caractérisé en ce qu'on fait réagir un agent électrophile nitrophénylique avec une polyfluoréthoxyphénylamine conformément au schéma suivant:

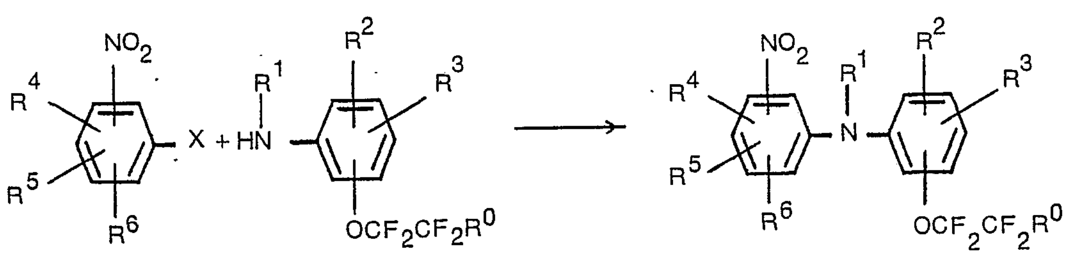

où $R^0$, $R^1$, $R^2$, $R^3$, $R^4$, $R^5$ et $R^6$ ont les significations définies dans la revendication 6 ci-dessus, tandis que X est un bon groupe qui s'éloigne tel qu'un atome d'halogène.

4. Procédé suivant la revendication 1, caractérisé en ce que l'agent électrophile phénylique substitué est le chlorure de tétra- ou penta-fluoréthoxyphényle.

5. Procédé suivant l'une quelconque des revendications 1 à 4 pour la préparation d'un composé répondant à la formule:

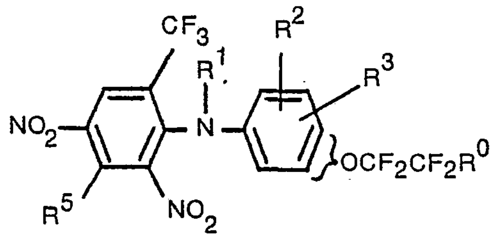

II

dans laquelle

$R^0$ représente un atome d'hydrogène ou un atome de fluor;

$R^1$ représente un atome d'hydrogène ou un groupe alkyle en $C_1$—$C_2$;

$R^2$ et $R^3$ représentent chacun indépendamment l'un de l'autre un atome d'hydrogène ou un atome d'halogène;

$R^5$ représente un atome d'hydrogène ou un atome d'halogène;

ainsi que des sels physiologiquement acceptables de ce composé.

6. Procédé de préparation d'un composé de formule I dans laquelle $R^1$ représente un groupe alkyle en $C_1$—$C_2$, caractérisé en ce qu'il consiste à alkyler un composé de formule I dans laquelle $R^1$ représente un atome d'hydrogène.

7. Procédé de préparation d'un composé de formule I dans laquelle $R^4$ représente un groupe alcoxy(en $C_1$—$C_4$)carbonyle, caractérisé en ce qu'il consiste à estérifier un composé de formule I dans laquelle $R^4$ représente un groupe hydroxycarbonyle.

8. Procédé de préparation d'un composé de formule I dans laquelle $R^2$ et $R^3$ représentent chacun indépendamment l'un de l'autre un atome d'halogène, caractérisé en ce qu'il consiste à halogéner un composé de formule I dans laquelle $R^2$ et $R^3$ représentent chacun un atome d'hydrogène.

9. Procédé de préparation des sels physiologiquement acceptables de formule I, caractérisé en ce qu'il consiste à faire réagir un composé de formule I dans laquelle $R^1$ représente un atome d'hydrogène, avec une base.

10. Procédé suivant la revendication 1, caractérisé en ce que le composé préparé est la N-(2,4-dinitro-6-trifluorométhylphényl)-2,4-dibromo-5-(1,1,2,2-tétrafluoréthoxy)benzénamine.

11. Procédé suivant la revendication 1, caractérisé en ce que le composé préparé est la N-(2,6-dinitro-4-trifluorométhylphényl)-2,4-dichloro-5-(1,1,2,2-tétrafluoréthoxy)benzénamine.

12. Procédé suivant la revendication 1, caractérisé en ce que le composé préparé est la N-(2,4-dinitro-6-trifluorométhylphényl)-4-(1,1,2,2 -tétrafluoréthoxy)benzénamine.

13. Formulation utile pour le traitement des infections fongiques du sol et des plantes, caractérisée en ce qu'elle comprend une quantité antifongique d'un composé défini dans l'une quelconque des revendications 1, 5, 10 et 11 ou d'un sel physiologiquement acceptable de ce composé, ainsi qu'un support approprié pour ce composé ou ses sels.

14. Formulation suivant la revendication 13, caractérisée en ce que l'ingrédient actif est la N-(2,4-dinitro-6-trifluorométhylphényl)-3-(1,1,2,2 -tétrafluoréthoxy)benzénamine.

15. Formulation suivant la revendication 13, caractérisée en ce que l'ingrédient actif est la N-(2-trifluorométhyl-4-nitrophényl)-3-(1,1,2,2 -tétrafluoréthoxy)benzénamine.

16. Procédé pour le traitement des infections fongiques des plantes et du sol, caractérisé en ce qu'il consiste à appliquer, à l'endroit à traiter, une quantité antifongique d'un composé défini dans l'une quelconque des revendications 1, 5, 10, 11, 14 et 15, ou d'un sel physiologiquement acceptable de ce composé.

17. Formulation anticoccidienne, caractérisée en ce qu'elle comprend une benzénamine anticoccidienne telle que définie dans la revendication 1, ainsi qu'un support approprié pour cette benzénamine.

18. Composition insecticide ou ectoparasiticide, caractérisée en ce qu'elle comprend un composé tel que défini dans la revendication 1, ainsi qu'un support approprié pour ce composé.

**Patentansprüche für die Vertragsstaaten: BE CH DE FR GB IT LI LU NL SE**

1. Verbindung der Formel

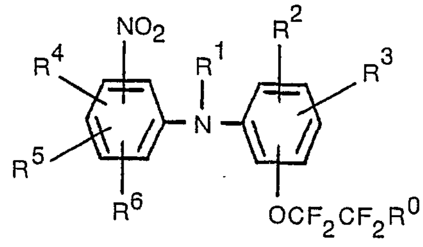

I

worin
$R^0$ Wasserstoff oder Fluor ist,
$R^1$ Wasserstoff oder $C_1$—$C_2$-Alkyl ist,
$R^2$ und $R^3$ unabhängig Wasserstoff oder Halogen sind,
$R^4$ Wasserstoff, Trifluormethyl, Cyano, $C_1$—$C_4$-Alkyl, Hydroxycarbonyl oder $C_1$—$C_4$-Alkoxycarbonyl ist,
$R^5$ Wasserstoff, Halogen, Nitro, Hydroxy, Methoxy oder Amino ist und
$R^6$ Wasserstoff oder Nitro ist,
und die physiologisch annehmbaren Salze hiervon.

2. Verbindung nach Anspruch 1, dadurch gekennzeichnet, daß sie die Formel

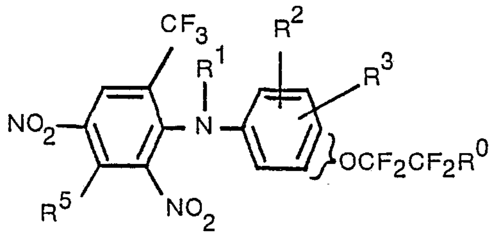

II

hat, worin
$R^0$ Wasserstoff oder Fluor ist,
$R^1$ Wasserstoff oder $C_1$—$C_2$-Alkyl ist,
$R^2$ und $R^3$ unabhängig Wasserstoff oder Halogen sind und
$R^5$ Wasserstoff oder Halogen ist,
und die physiologisch annehmbaren Salze hiervon.

3. Verbindung nach Anspruch 1, dadurch gekennzeichnet, daß diese Verbindung N-(2,4-Dinitro-6-trifluormethylphenyl)-2,4-dibrom-5-(1,1,2,2-tetrafluorethoxy)benzolamin ist.

4. Verbindung nach Anspruch 1, dadurch gekennzeichnet, daß diese Verbindung N-(2,6-Dinitro-4-trifluormethylphenyl)-2,4-dichlor-5-(1,1,2,2-tetrafluorethoxy)benzolamin ist.

5. Verbindung nach Anspruch 1, dadurch gekennzeichnet, daß diese Verbindung N-(2,4-Dinitro-6-trifluormethylphenyl)-4-(1,1,2,2-tetrafluorethoxy)benzolamin ist.

6. Verfahren zur Herstellung einer Verbindung der Formel

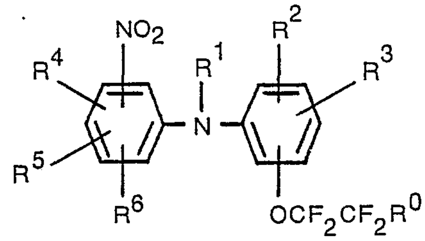

I

worin
$R^0$ Wasserstoff oder Fluor ist,
$R^1$ Wasserstoff oder $C_1$—$C_2$-Alkyl ist,
$R^2$ und $R^3$ unabhängig Wasserstoff oder Halogen sind,
$R^4$ Wasserstoff, Trifluormethyl, Cyano, $C_1$—$C_4$-Alkyl, Hydroxycarbonyl oder $C_1$—$C_4$-Alkoxycarbonyl ist,
$R^5$ Wasserstoff, Halogen, Nitro, Hydroxy, Methoxy oder Amino ist, und
$R^6$ Wasserstoff oder Nitro ist,
und der physiologisch annehmbaren Salze hiervon, dadurch gekennzeichnet, daß man in einem nichtreaktionsfähigen organischen Lösungsmittel in Gegenwart einer starken Base etwa eine äquimolare Menge einer elekrophilen substituierten Phenylverbindung mit einem Phenylaminderivat umsetzt.

7. Verfahren nach Anspruch 1, dadurch gekennzeichnet, daß man eine elektrophile Polyfluorethoxyphenylverbindung, wie ein Phenylhalogenid, mit einem Nitrophenylamin nach folgendem Reaktionsschema umsetzt:

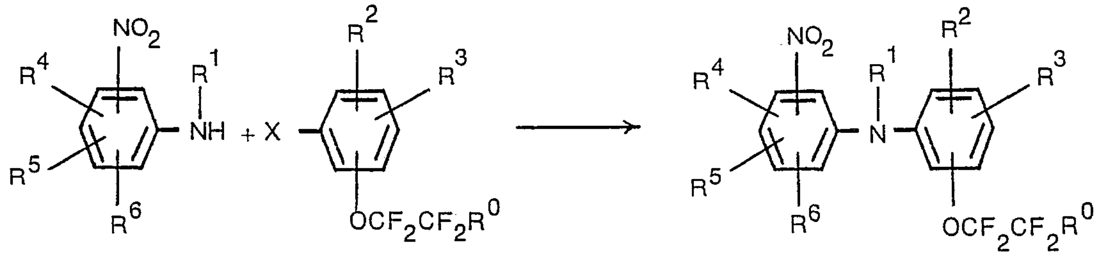

worin $R^0$, $R^1$, $R^2$, $R^3$, $R^4$, $R^5$ und $R^6$ wie in Anspruch 1 definiert sind und X eine gute Abgangsgruppe, wie Halogen, ist.

8. Verfahren nach Anspruch 6, dadurch gekennzeichnet, daß man eine elektrophile Nitrophenylverbindung mit einem Polyfluorethoxyphenylamin nach folgendem Reaktionsschema umsetzt:

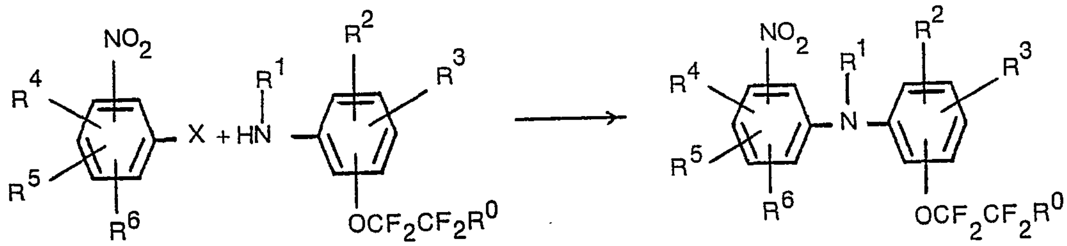

worin $R^0$, $R^1$, $R^2$, $R^3$, $R^4$, $R^5$ und $R^6$ wie in Anspruch 1 definiert sind und X eine gute Abgangsgruppe, wie Halogen, ist.

9. Verfahren nach Anspruch 6, dadurch gekennzeichnet, daß man als elektrophile substituierte Phenylverbindung Tetra- oder Pentafluorethoxyphenylchlorid verwendet.

10. Verfahren nach einem der Ansprüche 6 bis 9, dadurch gekennzeichnet, daß man eine Verbindung der Formel

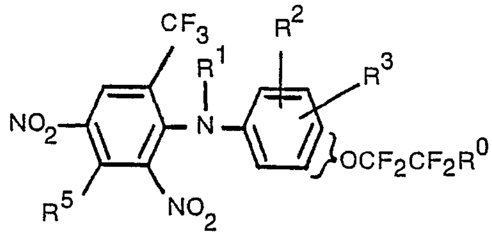

II

worin
$R^0$ Wasserstoff oder Fluor ist,
$R^1$ Wasserstoff oder $C_1$—$C_2$-Alkyl ist,
$R^2$ und $R^3$ unabhängig Wasserstoff oder Halogen sind, und
$R^5$ Wasserstoff oder Halogen ist,
oder ein physiologisch annehmbares Salz hiervon herstellt.

11. Verfahren zur Herstellung einer Verbindung der Formel I, worin $R^1$ für $C_1$—$C_2$-Alkyl steht, dadurch gekennzeichnet, daß man eine Verbindung der Formel I, worin $R^1$ Wasserstoff ist, alkyliert.

12. Verfahren zur Herstellung einer Verbindung der Formel I, worin $R^4$ für $C_1$—$C_4$-Alkoxycarbonyl steht, dadurch gekennzeichnet, daß man eine Verbindung der Formel I worin $R^4$ Hydroxycarbonyl ist, verestert.

13. Verfahren zur Herstellung einer Verbindung der Formel I, worin $R^2$ und $R^3$ unabhängig Halogen bedeuten, dadurch gekennzeichnet, daß man eine Verbindung der Formel I, worin $R^2$ und $R^3$ Wasserstoff sind, halogeniert.

14. Verfahren zur Herstellung physiologisch annehmbarer Salze von Verbindungen der Formel I, dadurch gekennzeichnet, daß man eine Verbindung der Formel I, worin $R^1$ Wasserstoff ist, mit einer Base umsetzt.

15. Verfahren nach Anspruch 6, dadurch gekennzeichnet, daß die hergestellte Verbindung in einem der Ansprüche 3, 4 oder 5 definiert ist.

16. Antikokzidiale Formulierung, dadurch gekennzeichnet, daß sie ein antikokzidiales Benzolamin der Formel

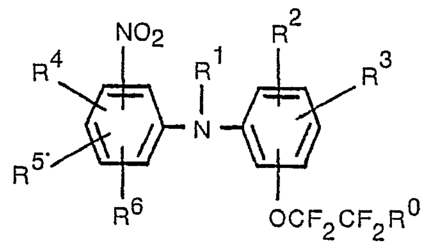

I

worin
$R^0$ Wasserstoff oder Fluor ist,
$R^1$ Wasserstoff oder $C_1$—$C_2$-Alkyl ist,
$R^2$ und $R^3$ unabhängig Wasserstoff oder Halogen sind,

$R^4$ Wasserstoff, Trifluormethyl, Cyano, $C_1$—$C_4$-Alkyl, Hydroxycarbonyl oder $C_1$—$C_4$-Alkoxycarbonyl ist,

$R^5$ Wasserstoff, Halogen, Nitro, Hydroxy, Methoxy oder Amino ist, und

$R^6$ Wasserstoff oder Nitro ist,

oder die physiologisch annehmbaren Salze hiervon zusammen mit einem geeigneten Träger hierfür enthält.

17. Formulierung nach Anspruch 16, dadurch gekennzeichnet, daß der Wirkstoff die Formel

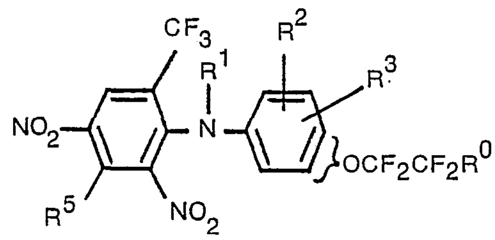

II

hat, worin

$R^0$ Wasserstoff oder Fluor ist,

$R^1$ Wasserstoff oder $C_1$—$C_2$-Alkyl ist,

$R^2$ und $R^3$ unabhängig Wasserstoff oder Halogen sind und

$R^5$ Wasserstoff oder Halogen ist,

oder ein physiologisch annehmbares Salz hiervon ist.

18. Formulierung nach Anspruch 16 oder 17, dadurch gekennzeichnet, daß der Wirkstoff N-(2,4-Dinitro-6-trifluormethylphenyl)-4-(1,1,2,2-tetrafluorethoxy)benzolamin ist.

19. Formulierung nach Anspruch 16, 17 oder 18, dadurch gekennzeichnet, daß die Kombination ein Futtermittel für Geflügel ist.

20. Formulierung nach Anspruch 16, 17 oder 18, dadurch gekennzeichnet, daß die Kombination ein Vorfuttermittel für Geflügel ist.

21. Formulierung zur Behandlung von Pilzinfektionen im Boden und auf Pflanzen, dadurch gekennzeichnet, daß sie eine antifungale Menge einer Verbindung der Formel

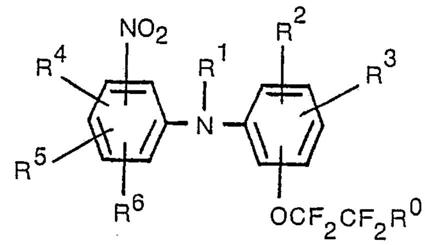

I

worin

$R^0$ Wasserstoff oder Fluor ist,

$R^1$ Wasserstoff oder $C_1$—$C_2$-Alkyl ist,

$R^2$ und $R^3$ unabhängig Wasserstoff oder Halogen sind,

$R^4$ Wasserstoff, Trifluormethyl, Cyano, $C_1$—$C_4$-Alkyl, Hydroxycarbonyl oder $C_1$—$C_4$-Alkoxycarbonyl ist,

$R^5$ Wasserstoff, Halogen, Nitro, Hydroxy, Methoxy oder Amino ist, und

$R^6$ Wasserstoff oder Nitro ist,

oder ein physiologisch annehmbares Salz hiervon und einen geeigneten Träger hierfür enthält..

22. Formulierung nach Anspruch 21, dadurch gekennzeichnet, daß der Wirkstoff in einem der Ansprüche 2, 3 oder 4 definiert ist.

23. Formulierung nach Anspruch 21, dadurch gekennzeichnet, daß der Wirkstoff N-(2,4-Dinitro-6-trifluormethylphenyl)-3-(1,1,2,2-tetrafluorethoxy)benzolamin ist.

24. Formulierung nach Anspruch 21, dadurch gekennzeichnet, daß der Wirkstoff N-(2-Trifluormethyl-4-nitrophenyl)-3-(1,1,2,2-tetrafluorethoxy)benzolamin ist.

25. Verfahren zur Behandlung von Pilzinfektionen bei Pflanzen und im Boden, dadurch gekennzeichnet, daß man auf die zu behandelnde Stelle eine antifungale Menge einer Verbindung der Formel

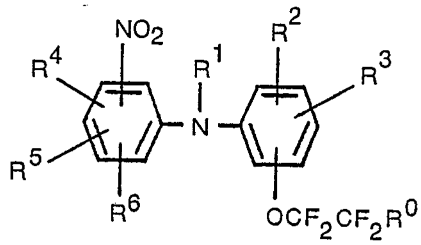

I

worin

$R^0$ Wasserstoff oder Fluor ist,

$R^1$ Wasserstoff oder $C_1$—$C_2$-Alkyl ist,

$R^2$ und $R^3$ unabhängig Wasserstoff oder Halogen sind,

$R^4$ Wasserstoff, Trifluormethyl, Cyano, $C_1$—$C_4$-Alkyl, Hydroxycarbonyl oder $C_1$—$C_4$-Alkoxycarbonyl ist,

$R^5$ Wasserstoff, Halogen, Nitro, Hydroxy, Methoxy oder Amino ist, und

$R^6$ Wasserstoff oder Nitro ist,

oder ein physiologisch annehmbares Salz hiervon aufbringt.

26. Verfahren nach Anspruch 25, dadurch gekennzeichnet, daß die aufgebrachte wirksame Verbindung in einem der Ansprüche 2, 3 oder 4 definiert ist.

27. Verfahren nach Anspruch 25, dadurch gekennzeichnet, daß die aufgebrachte wirksame Verbindung N-(2,4-Dinitro-6-trifluormethylphenyl)-3-(1,1,2,2-tetrafluorethoxy)benzolamin ist.

28. Verfahren nach Anspruch 25, dadurch gekennzeichnet, daß die aufgebrachte wirksame Verbindung N-(2-Trifluormethyl-4-nitrophenyl)-3-(1,1,2,2-tetrafluorethoxy)benzolamin ist.

29. Insektizide oder ektoparasitizide Zusammensetzung, dadurch gekennzeichnet, daß sie eine Verbindung gemäß Anspruch 1 in Verbindung mit einem geeigneten Träger hierfür enthält.

**Patentansprüche für den Vertragsstaat: AT**

1. Verfahren zur Herstellung einer Verbindung der Formel I

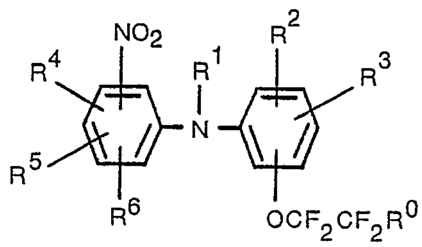

I

worin

$R^0$ Wasserstoff oder Fluor ist,

$R^1$ Wasserstoff oder $C_1$—$C_2$-Alkyl ist,

$R^2$ und $R^3$ unabhängig Wasserstoff oder Halogen sind,

$R^4$ Wasserstoff, Trifluormethyl, Cyano, $C_1$—$C_4$-Alkyl, Hydroxycarbonyl oder $C_1$—$C_4$-Alkoxycarbonyl ist,

$R^5$ Wasserstoff, Halogen, Nitro, Hydroxy, Methoxy oder Amino ist, und

$R^6$ Wasserstoff oder Nitro ist,

und der physiologisch annehmbaren Salze hiervon, dadurch gekennzeichnet, daß man in einem nichtreaktionsfähigen organischen Lösungsmittel in Gegenwart einer starken Base etwa eine äquimolare Menge einer elektrophilen substituierten Phenylverbindung mit einem Phenylaminderivat umsetzt.

2. Verfahren nach Anspruch 1, dadurch gekennzeichnet, daß man eine elektrophile Polyfluorethoxyphenylverbindung, wie ein Phenylhalogenid, mit einem Nitrophenylamin nach folgendem Reaktionsschema umsetzt:

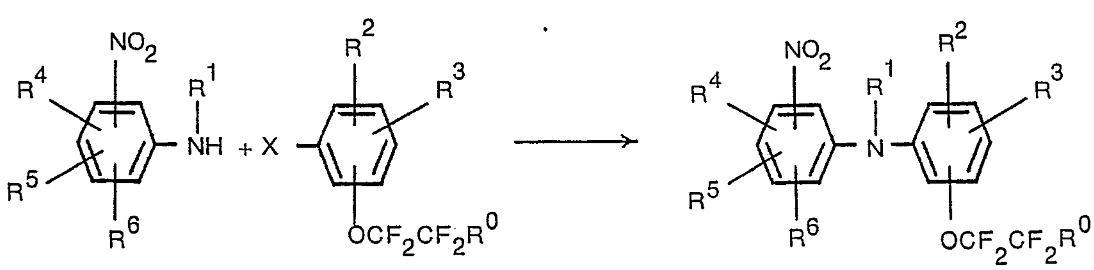

worin $R^0$, $R^1$, $R^2$, $R^3$, $R^4$, $R^5$ und $R^6$ wie in Anspruch 1 definiert sind und X eine gute Abgangsgruppe, wie Halogen, ist.

3. Verfahren nach Anspruch 1, dadurch gekennzeichnet, daß man eine elektrophile Nitrophenylverbindung mit einem Polyfluorethoxyphenylamin nach folgendem Reaktionsschema umsetzt:

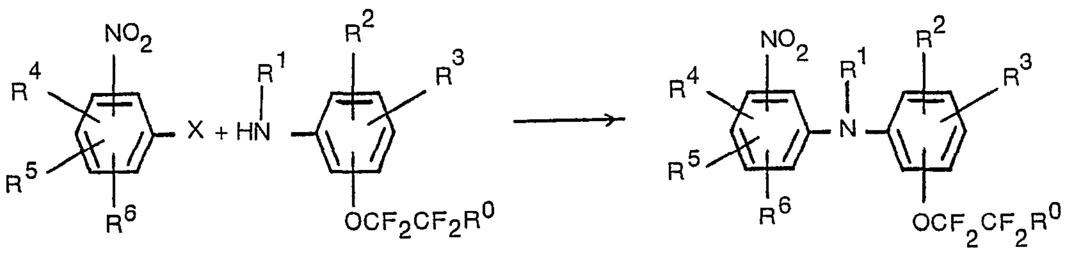

worin $R^0$, $R^1$, $R^2$, $R^3$, $R^4$, $R^5$ und $R^6$ wie in Anspruch 1 definiert sind und X eine gute Abgangsgruppe, wie Halogen, ist.

4. Verfahren nach Anspruch 1, dadurch gekennzeichnet, daß man als elektrophile substituierte Phenylverbindung Tetra- oder Pentafluorethoxyphenylchlorid verwendet.

5. Verfahren nach einem der Ansprüche 1 bis 4, dadurch gekennzeichnet, daß man eine Verbindung der Formel

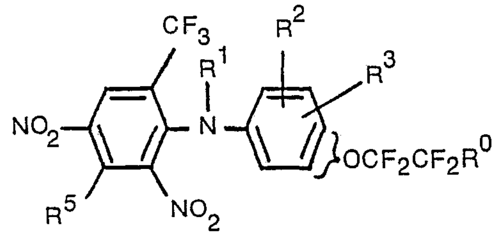

II

worin
$R^0$ Wasserstoff oder Fluor ist,
$R^1$ Wasserstoff oder $C_1$—$C_2$-Alkyl ist,
$R^2$ und $R^3$ unabhängig Wasserstoff oder Halogen sind, und
$R^5$ Wasserstoff oder Halogen ist,
oder ein physiologisch annehmbares Salz hiervon herstellt.

6. Verfharen zur Herstellung einer Verbindung der Formel I, worin $R^1$ für $C_1$—$C_2$-Alkyl steht, dadurch gekennzeichnet, daß man eine Verbindung der Formel I, worin $R^1$ Wasserstoff ist, alkyliert.

7. Verfahren zur Herstellung einer Verbindung der Formel I, worin $R^4$ für $C_1$—$C_4$-Alkoxycarbonyl steht, dadurch gekennzeichnet, daß man eine Verbindung der Formel I, worin $R^4$ Hydroxycarbonyl bedeutet, verestert.

8. Verfahren zur Herstellung einer Verbindung der Formel I, worin $R^2$ und $R^3$ unabhängig Halogen bedeuten, dadurch gekennzeichnet, daß man eine Verbindung der Formel I, worin $R^2$ und $R^3$ Wasserstoff sind, halogeniert.

9. Verfahren zur Herstellung physiologisch annehmbarer Salze von Verbindung der Formel I, dadurch gekennzeichnet, daß man eine Verbindung der Formel I, worin $R^1$ Wasserstoff ist, mit einer Base umsetzt.

10. Verfahren nach Anspruch 1, dadurch gekennzeichnet, daß man als Verbindung N-(2,4-Dinitro-6-trifluormethylphenyl)-2,4-dibrom-5-(1,1,2,2-tetrafluorethoxy)benzolamin herstellt.

11. Verfahren nach Anspruch 1, dadurch gekennzeichnet, daß man als Verbindung N-(2,6-Dinitro-4-trifluormethylphenyl)-2,4-dichlor-5-(1,1,2,2-tetrafluorethoxy)benzolamin herstellt.

12. Verfahren nach Anspruch 1, dadurch gekennzeichnet, daß man als Verbindung N-(2,4-Dinitro-6-trifluormethylphenyl)-4-(1,1,2,2-tetrafluorethoxy)benzolamin herstellt.

13. Formulierung zur Behandlung von Pilzinfektionen im Boden und bei Pflanzen, dadurch gekennzeichnet, daß sie eine antifungale Menge einer Verbindung nach einem der Ansprüche 1, 5, 10 oder 11 oder ein physiologisch annehmbares Salz hiervon und einen geeigneten Träger hierfür enthält.

14. Formulierung nach Anspruch 13, dadurch gekennzeichnet, daß sie als Wirkstoff N-(2,4-Dinitro-6-trifluormethylphenyl)-3-(1,1,2,2-tetrafluorethoxy)benzolamin enthält.

15. Formulierung nach Anspruch 13, dadurch gekennzeichnet, daß sie als Wirkstoff N-(2-Trifluormethyl-4-nitrophenyl)-3-(1,1,2,2-tetrafluorethoxy)benzolamin enthält.

16. Verfahren zur Behandlung von Pilzinfektionen bei Pflanzen und beim Boden, dadurch gekennzeichnet, daß man auf die zu behandelnde Stelle eine antifungale Menge einer Verbindung nach einem der Ansprüche 1, 5, 10, 11, 14 oder 15 oder ein physiologisch annehmbares Salz hiervon aufbringt.

17. Antikokzidiale Formulierung, dadurch gekennzeichnet, daß sie ein antikokzidiales Benzolamin gemäß Anspruch 1 und einen geeigneten Träger hierfür enthält.

18. Insektizide oder ektoparasitizide Zusammensetzung, dadurch gekennzeichnet, daß sie eine Verbindung gemäß Anspruch 1 und einen geeigneten Träger hierfür enthält.